(19) Europäisches Patentamt
European Patent Office
Office européen des brevets

(11) **EP 3 571 976 B1**

(12) **EUROPEAN PATENT SPECIFICATION**

(45) Date of publication and mention
of the grant of the patent:
**22.02.2023 Bulletin 2023/08**

(21) Application number: **19170125.9**

(22) Date of filing: **18.04.2019**

(51) International Patent Classification (IPC):
**A61B 1/06** *(2006.01)*     **A61B 1/00** *(2006.01)*
**A61B 5/107** *(2006.01)*

(52) Cooperative Patent Classification (CPC):
**A61B 1/00009; A61B 1/0623; A61B 1/0638;**
**A61B 5/1076; A61B 5/1079**

(54) **ENDOSCOPE APPARATUS**

ENDOSKOPVORRICHTUNG

APPAREIL D'ENDOSCOPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.04.2018 JP 2018081223**

(43) Date of publication of application:
**27.11.2019 Bulletin 2019/48**

(73) Proprietor: **FUJIFILM Corporation
Tokyo 106-8620 (JP)**

(72) Inventor: **YANO, Takashi
Kanagawa (JP)**

(74) Representative: **Klunker IP
Patentanwälte PartG mbB
Destouchesstraße 68
80796 München (DE)**

(56) References cited:
**WO-A1-2018/051679     JP-A- 2016 106 867**

**Description**

**BACKGROUND OF THE INVENTION**

1. Field of the Invention

The present invention relates to an endoscope apparatus that measures the size of a subject.

2. Description of the Related Art

A distance to an object to be observed, the size of an object to be observed, or the like is measured in an endoscope apparatus. For example, JP1992-012724A (JP-H04-012724A) discloses an endoscope apparatus that obtains the three-dimensional information of an object to be observed irradiated with planar light by sweeping planar light from the distal end of an endoscope and processing the taken image of the portion to be observed in a state where parallel light is swept. Further, JP2017-508529A discloses an endoscope apparatus that applies planar light to an object to be observed from the distal end of an endoscope and superimposes and displays a mesh, which shows the trajectory of the planar light, and a curved line where the planar light crosses an object to be observed on a taken image. In a case two points positioned on the curved line superimposed on the taken image are selected in this endoscope apparatus, a distance between the two points is calculated and displayed.

WO 2018/051679 A1 discloses an endoscope apparatus in which in a measurement mode, the illumination light is turned off or lowered to such a degree that the recognition of a measurement spot is not affected.

**SUMMARY OF THE INVENTION**

It is preferable that auxiliary measurement light for measurement having a wavelength in a wavelength range where directivity is high and reflectivity from an object to be observed is high is used to measure a distance to an object to be observed, the size of an object to be observed, or the like as described above. In a case where the color of an object to be measured included in the object to be observed is the same as the color of the auxiliary measurement light when illumination light, which is used to illuminate the object to be observed with brightness, and the auxiliary measurement light are simultaneously applied to the object to be observed, the visibility of the object to be measured may deteriorate. For example, since the measurement light is spread and superimposed on a red portion in a case where the object to be measured is the red portion and the auxiliary measurement light is light having a wavelength range corresponding to a red color, there is a case where it may be difficult to recognize the red portion.

An object of the invention is to provide an endoscope apparatus where the obstruction of the visibility of an object to be measured caused by auxiliary measurement light does not occur even though the auxiliary measurement light is used to measure the size and the like of the object to be measured included in an object to be observed.

An endoscope apparatus according to the invention as defined by claim 1 comprises a light source unit for illumination light that generates illumination light used to illuminate a subject, an auxiliary measurement light-emitting unit that emits auxiliary measurement light, a light source control unit that allows the illumination light to be continuously emitted and allows the auxiliary measurement light to be emitted in the form of a pulse at a specific frame interval, an imaging element that images the subject, a signal processing unit that generates a first taken image obtained through the imaging of the subject illuminated with the illumination light and generates a second taken image obtained through the imaging of the subject illuminated with the illumination light and the auxiliary measurement light, and a display control unit that allows a display unit to display a specific image where a measurement marker obtained from the second taken image is displayed in the first taken image.

It is preferable that the auxiliary measurement light-emitting unit is a first auxiliary measurement light-emitting unit emitting planar auxiliary measurement light as the auxiliary measurement light, and the measurement marker is a first measurement marker including a crossing line corresponding to a portion, which crosses the subject, of a plane formed by the planar auxiliary measurement light and gradations provided on the crossing line and serving as an index of a size of the subject. It is preferable that the endoscope apparatus further comprises an imaging optical system including an objective lens used to form an image of the subject on the imaging element, the auxiliary measurement light-emitting unit emits the auxiliary measurement light in a state where the plane crosses an optical axis of the objective lens, and the plane is included in an effective imaging range that is a range where an effective visual field predetermined in a visual field of the imaging optical system and a depth-of-field of the imaging optical system overlap with each other.

It is preferable that the auxiliary measurement light-emitting unit is a second auxiliary measurement light-emitting unit emitting spot-like auxiliary measurement light as the auxiliary measurement light, the signal processing unit includes a spot position recognition unit recognizing a position of a spot, which is a substantially circular area formed on the subject by the spot-like auxiliary measurement light, in the second taken image and a measurement marker generation unit including a second measurement marker representing an actual size of the subject as the measurement marker on the basis of the position of the spot in the second taken image, and the display control unit displays the second measurement marker in the first taken image. It is preferable that the display control unit displays a spot display portion, which corresponds to the position of the spot, in the first taken image in addition to the second measurement marker. It is preferable that the second measurement marker has any one of a cruciform shape, a cruciform shape with gradations, a distorted cruciform shape, a circular-and-cruciform shape, or a shape of a measurement point group. It is preferable that the second measurement marker has any one of a shape of a plurality of concentric circles, a shape of a plurality of color concentric circles, or a shape of a plurality of distorted concentric circles.

It is preferable that the imaging element is a global shutter-type imaging element performing exposure and reading of electric charges on each pixel at the same timing to output an image signal used to obtain the first taken image or the second taken image, and, until the first taken image is acquired at a second timing next to a first timing after the first taken image is acquired at the first timing, the first taken image acquired at the first timing is continuously displayed and the second taken image is not displayed in the specific image.

It is preferable that the imaging element is a rolling shutter-type imaging element including a plurality of lines used to image an object to be observed illuminated with the illumination light or the auxiliary measurement light, performing exposure at different exposure timings for the respective lines, and reading electric charges at different reading timings for the respective lines to output an image signal used to obtain the first taken image or the second taken image, and, until the first taken image is acquired at a second timing next to a first timing after the first taken image is acquired at the first timing, the first taken image acquired at the first timing is continuously displayed and the second taken image is not displayed in the specific image.

It is preferable that the imaging element is a rolling shutter-type imaging element including a plurality of lines used to image an object to be observed illuminated with the illumination light or the auxiliary measurement light, performing exposure at different exposure timings for the respective lines, and reading electric charges at different reading timings for the respective lines to output an image signal used to obtain the first taken image or the second taken image, the rolling shutter-type imaging element provides a blanking period in which the output of the image signal is prohibited, the light source control unit emits the auxiliary measurement light in the blanking period, and, until the first taken image is acquired at a second timing next to a first timing after the first taken image is acquired at the first timing, the first taken image acquired at the first timing is continuously displayed and the second taken image is not displayed in the specific image.

It is preferable that a first mode in which the first taken image is displayed on the display unit and a second mode in which the specific image is displayed on the display unit are provided, and a reading period of the imaging element in the second mode is set shorter than a reading period of the imaging element in the first mode. It is preferable that a frame rate of the display unit, which displays the specific image, is equal to a value of a sum of the reading period of the imaging element in the second mode and the blanking period. It is preferable that a first mode in which the first taken image is displayed on the display unit and a second mode in which the specific image is displayed on the display unit are provided, and a reading period of the imaging element in the first mode is set equal to a reading period of the imaging element in the second mode. It is preferable that a value of a sum of the reading period of the imaging element in the first mode and the blanking period is equal to a value of a sum of the reading period of the imaging element in the second mode and the blanking period.

According to the aspect of the invention, the obstruction of the visibility of an object to be measured caused by auxiliary measurement light does not occur even though the auxiliary measurement light is used to measure the size and the like of the object to be measured.

## BRIEF DESCRIPTION OF THE DRAWINGS

Fig. 1 is a diagram showing the appearance of an endoscope apparatus.
Fig. 2 is a plan view of a distal end portion of an endoscope.
Fig. 3 is a block diagram showing the function of the endoscope apparatus.
Fig. 4 is a block diagram of a first auxiliary measurement light-emitting unit.
Fig. 5 is a diagram illustrating a relationship between a distal end portion of an endoscope of a first embodiment and a visual field 21A and an effective imaging range 21C in a depth-of-field R1.
Fig. 6 is a diagram illustrating a relationship between the visual field 21A and the effective imaging range 21C in the depth-of-field R1 and a plane 30F that is formed by auxiliary measurement light.
Fig. 7 is a diagram illustrating an optical image OP1.
Fig. 8 is a diagram illustrating an optical image OP2.
Fig. 9 is a diagram illustrating an optical image OP3.

Fig. 10 is an image diagram showing a state where a polyp P is present at a position corresponding to a distance L1.

Fig. 11 is an image diagram showing a state where a polyp P is present at a position corresponding to a distance larger than the distance L1.

Fig. 12 is an image diagram showing a state where a polyp P is present at a position corresponding to a distance smaller than the distance L1.

Fig. 13 is an image diagram of a specific image that includes a frame 70B showing an effective visual field 21B.

Fig. 14 is an image diagram of a specific image where gradations 70A are not displayed in a case where the entire crossing line is positioned outside a range 21X.

Fig. 15 is an image diagram of a specific image where gradations 70A are not displayed in a case where a part of the crossing line is positioned outside a range 21X.

Fig. 16 is a plan view showing the distal end portion of the endoscope that includes an attachable and detachable auxiliary measurement light-emitting unit.

Fig. 17 is a diagram illustrating emission patterns of illumination light and auxiliary measurement light in a length measurement mode.

Fig. 18 is a diagram illustrating a first pattern of the length measurement mode.

Fig. 19 is a diagram illustrating a second pattern of the length measurement mode.

Fig. 20 is a diagram illustrating a third pattern of the length measurement mode.

Fig. 21 is a diagram illustrating a fourth pattern of the length measurement mode.

Fig. 22 is a diagram illustrating a fifth pattern of the length measurement mode.

Fig. 23 is a block diagram of a second auxiliary measurement light-emitting unit.

Fig. 24 is a diagram illustrating a relationship between a distal end portion of an endoscope of a second embodiment and a near end Px, an intermediate vicinity Py, and a far end Pz in a range Rx of an observation distance.

Fig. 25 is a block diagram showing the function of a signal processing unit of the second embodiment.

Fig. 26 is an image diagram showing a spot display portion and a second measurement marker in a case where an observation distance corresponds to the near end Px.

Fig. 27 is an image diagram showing a spot display portion and a second measurement marker in a case where an observation distance corresponds to the intermediate vicinity Py.

Fig. 28 is an image diagram showing a spot display portion and a second measurement marker in a case where an observation distance corresponds to the far end Pz.

Fig. 29 is a diagram illustrating second measurement markers having a cruciform shape with gradations, a distorted cruciform shape, a circular-and-cruciform shape, and the shape of a measurement point group.

Fig. 30 is a diagram illustrating a graph paper-shaped chart that is used to measure a relationship between the position of a spot and the size of the second measurement marker in a case where an observation distance corresponds to the near end Px.

Fig. 31 is a diagram illustrating a graph paper-shaped chart that is used to measure a relationship between the position of a spot and the size of the second measurement marker in a case where an observation distance corresponds to the far end Py.

Fig. 32 is a graph showing a relationship between the pixel position of a spot in an X direction and the number of pixels of the second measurement marker in the X direction.

Fig. 33 is a graph showing a relationship between the pixel position of a spot in a Y direction and the number of pixels of the second measurement marker in the X direction.

Fig. 34 is a graph showing a relationship between the pixel position of a spot in the X direction and the number of pixels of the second measurement marker in the Y direction.

Fig. 35 is a graph showing a relationship between the pixel position of a spot in the Y direction and the number of pixels of the second measurement marker in the Y direction.

Fig. 36 is an image diagram showing a marker that has the shape of three concentric circles having the same color.

Fig. 37 is an image diagram showing a marker that has the shape of three concentric circles having different colors.

Fig. 38 is an image diagram showing a marker having the shape of distorted concentric circles.

## DESCRIPTION OF THE PREFERRED EMBODIMENTS

First embodiment

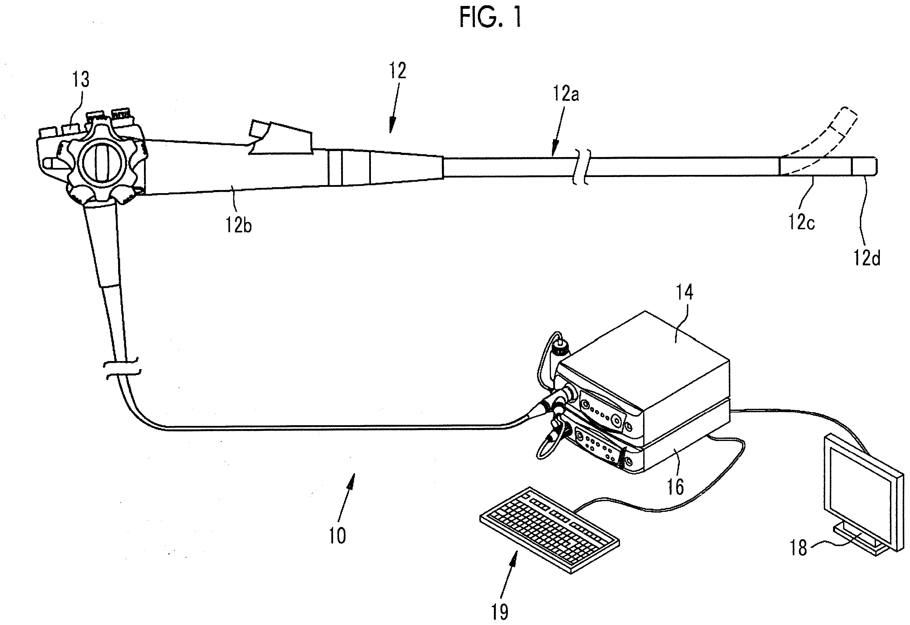

As shown in Fig. 1, an endoscope apparatus 10 includes an endoscope 12, a light source device 14, a processor device 16, a monitor 18, and a user interface 19. The endoscope 12 is optically connected to the light source device 14, and is electrically connected to the processor device 16. The processor device 16 is electrically connected to the monitor 18 (display unit) that displays an image. The user interface 19 is connected to the processor device 16, and is used for various setting operations and the like for the processor device 16. The user interface 19 includes a mouse and the like addition to a keyboard shown in Fig. 1.

The endoscope 12 includes an insertion part 12a that is to be inserted into a subject, an operation part 12b that is provided at a proximal end portion of the insertion part 12a, and a bendable portion 12c and a distal end portion 12d that are provided at a distal end of the insertion part 12a. The bendable portion 12c operates to be bent by the operation of an angle knob 12e of the operation part 12b. The distal end portion 12d is oriented in a desired direction by the bending operation of the bendable portion 12c.

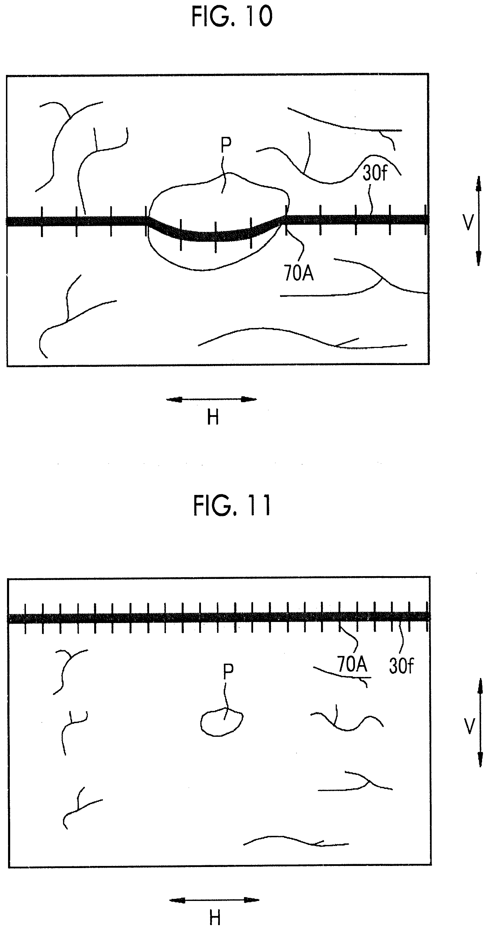

The endoscope 12 has a normal mode (first mode) and a length measurement mode (second mode), and these modes are switched by a mode changeover switch 13 that is provided on the operation part 12b of the endoscope 12. The normal mode is a mode where an object to be observed with illumination light is illuminated. In the length measurement mode, an object to be observed is illuminated with illumination light or planar auxiliary measurement light to be described later, so that gradations 70A used to measure a specific portion is displayed on the monitor 18. The gradations 70A are provided on a line referred to as a crossing line 30f (see Fig. 10 and the like). In the first embodiment, a linear measurement marker including the gradations 70A and the crossing line 30f corresponds to a first measurement marker used for measurement.

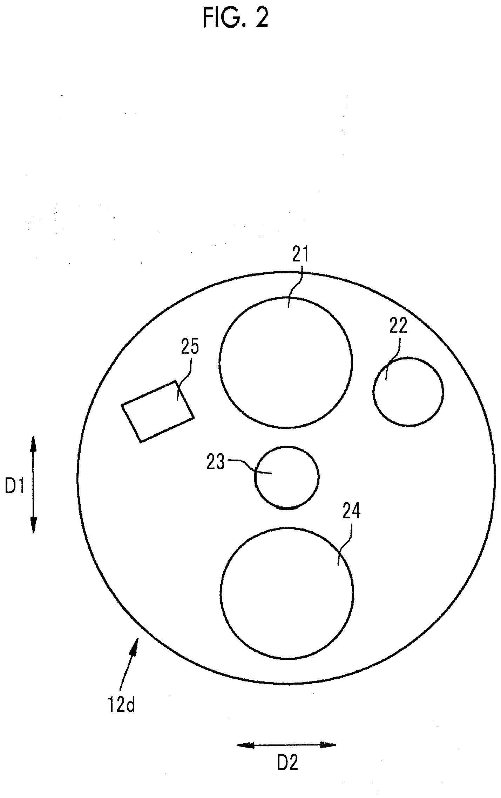

As shown in Fig. 2, the distal end portion of the endoscope 12 has a substantially circular shape; and is provided with an objective lens 21 that is positioned closest to a subject among optical members of an imaging optical system of the endoscope 12, an illumination lens 22 that is used to irradiate a subject with illumination light, an auxiliary measurement lens 23 that is used to illuminate a subject with planar auxiliary measurement light to be described later, an opening 24 that allows a treatment tool to protrude toward a subject, and an air/water supply nozzle 25 that is used to supply air and water.

An optical axis Ax of the objective lens 21 extends in a direction perpendicular to the plane of paper. A vertical first direction D1 is orthogonal to the optical axis Ax, and a horizontal second direction D2 is orthogonal to the optical axis Ax and the first direction D1. The objective lens 21 and the auxiliary measurement lens 23 are arranged in the first direction D1.

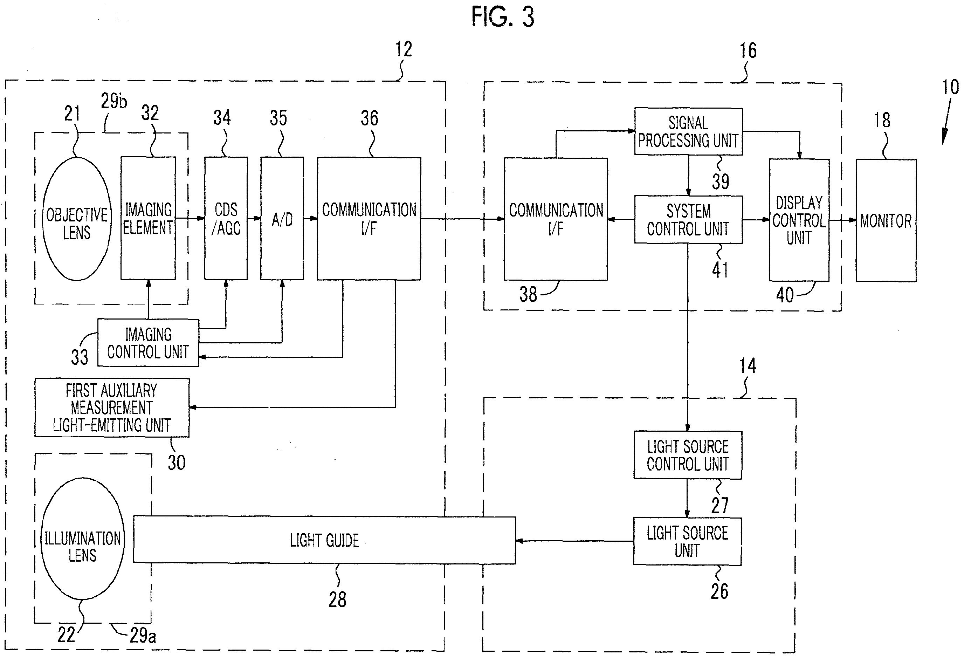

As shown in Fig. 3, the light source device 14 comprises a light source unit 26 (light source unit for illumination light) and a light source control unit 27. The light source unit 26 generates illumination light that is used to illuminate a subject. Illumination light emitted from the light source unit 26 is incident on a light guide 28, and is applied to a subject through the illumination lens 22. In the light source unit 26, a white light source emitting white light, a plurality of light sources, which includes a white light source and a light source emitting another color light (for example, a blue light source emitting blue light), or the like is used as a light source of illumination light. The light source control unit 27 is connected to a system control unit 41 of the processor device 16. The light source control unit 27 controls the light source unit on the basis of a command output from the system control unit 41.

The distal end portion 12d of the endoscope 12 is provided with an illumination optical system 29a, an imaging optical system 29b, and a first auxiliary measurement light-emitting unit 30. The illumination optical system 29a includes the illumination lens 22, and an object to be observed is irradiated with light, which is emitted from the light guide 28, through the illumination lens 22. The imaging optical system 29b includes the objective lens 21 and an imaging element 32. Light reflected from the object to be observed is incident on the imaging element 32 through the objective lens 21. Accordingly, the reflected image of the object to be observed is formed on the imaging element 32.

The imaging element 32 is a color imaging sensor, and takes the reflected image of a subject and outputs image signals. It is preferable that the imaging element 32 is a charge coupled device (CCD) imaging sensor, a complementary metal-oxide semiconductor (CMOS) imaging sensor, or the like. The imaging element 32 used in the invention is a color imaging sensor that is used to obtain RGB image signals corresponding to three colors of R (red), G (green), and B (blue). The imaging element 32 is controlled by an imaging control unit 33.

The image signals output through the imaging element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to the processor device 16 through a communication inter/face (I/F) 36.

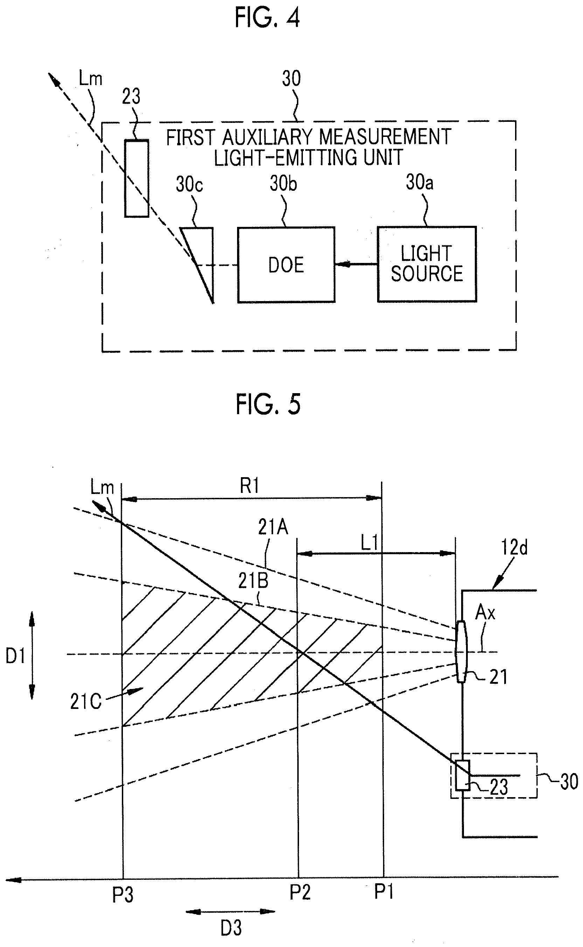

As shown in Fig. 4, the first auxiliary measurement light-emitting unit 30 comprises a light source 30a, a diffractive optical element (DOE) 30b, a prism 30c, and the auxiliary measurement lens 23. The light source 30a is to emit light having a color that can be detected by pixels of the imaging element 32 (specifically visible light), and includes a light-emitting element, such as a laser diode (LD) or a light-emitting diode (LED), and a condenser lens that condenses light emitted from the light-emitting element.

Light emitted from the light source 30a is red light having a wavelength of, for example, 650 nm, but is not limited to this wavelength. The light source 30a is controlled by the system control unit 41, and emits light on the basis of a command output from the system control unit 41. The DOE 30b converts the light, which is emitted from the light source, into auxiliary measurement light that is planar light. The converted planar auxiliary measurement light is parallel to the optical axis Ax of the objective lens 21.

The prism 30c is an optical member that is used to change the travel direction of planar auxiliary measurement light converted by the DOE 30b. The prism 30c changes the travel direction of planar auxiliary measurement light so that planar auxiliary measurement light crosses the visual field of the imaging optical system including the objective lens 21 and lens groups. A subject is irradiated with planar auxiliary measurement light Lm, which is emitted from the prism 30c, through the auxiliary measurement lens 23.

The first auxiliary measurement light-emitting unit 30 has only to be capable of emitting planar light toward the visual field of the imaging optical system. For example, the light source 30a may be provided in the light source device and light emitted from the light source 30a may be guided to the DOE 30b by optical fibers. Further, the prism 30c may not be used and the directions of the light source 30a and the DOE 30b may be inclined with respect to the optical axis Ax so that planar auxiliary measurement light Lm is emitted in a direction crossing the visual field of the imaging optical system.

As shown in Fig. 3, the processor device 16 comprises a communication inter/face (I/F) 38 that is connected to the communication I/F of the endoscope, a signal processing unit 39, a display control unit 40, and a system control unit 41. The communication I/F receives the image signals, which are transmitted from the communication I/F 36 of the endoscope 12, and transmits the image signals to the signal processing unit 39. A memory, which temporarily stores the image signals received from the communication I/F, is built in the signal processing unit 39, and the signal processing unit 39 processes an image signal group, which is a set of the image signals stored in the memory, to generate a taken image. The display control unit 40 displays the taken image, which is generated by the signal processing unit 39, on the monitor 18. The system control unit 41 controls the imaging element 32 through the imaging control unit 33 that is provided in the endoscope 12. The imaging control unit 33 also controls the CDS/AGC circuit 34 and the A/D converter 35 according to the control of the imaging element 32. Further, the system control unit 41 controls the light source unit 26 through the light source control unit 27. Furthermore, the system control unit 41 controls the light source 30a of the first auxiliary measurement light-emitting unit 30.

A method of displaying the gradations 70A on the crossing line 30f in the length measurement mode will be described below. As shown in Fig. 5, the imaging optical system including the objective lens 21 has a visual field 21A that is shown by a dotted line of Fig. 5. The imaging element 32 can image a subject that is positioned in the visual field 21A. The visual field 21A has a circular shape on the cross section thereof perpendicular to the optical axis Ax. A depth-of-field where a subject is brought into focus is present in the imaging optical system including the objective lens 21. A depth-of-field R1 is formed of a range between a position P1 and a position P3 in an optical axis direction D3.

The depth-of-field R1 is optionally determined, but the imaging optical system is designed in the endoscope so that the depth-of-field R1 is present in a range where a distance from the objective lens 21 is 3 mm or more and 100 mm or less. The position P1 is present at a position where a distance from the distal end portion of the objective lens 21 (a point on a distal end closest to a subject in a direction along the optical axis Ax of the objective lens 21) is, for example, 3 mm. The position P3 is present at a position where a distance from the distal end portion of the objective lens 21 is, for example, 100 mm. Accordingly, the imaging element 32 can image a subject, which is positioned in the visual field 21A and the depth-of-field R1, with high resolution.

The visual field 21A is a field having an angle of view in the range of, for example, 140° to 170°. As described above, the visual field 21A is set wide in the endoscope 12. For this reason, distortion appears around the visual field 21A in the optical image of a subject that is formed on the light-receiving surface of the imaging element 32 by the imaging optical system.

An effective visual field 21B shown by a broken line is predetermined in the endoscope apparatus 10 as a range, which does not substantially cause distortion to appear in the optical image, of the visual field 21A. The effective visual field 21B serves as a range that is suitable to display gradations as the index of a subject to be described later. A range where the effective visual field 21B and the depth-of-field R1 overlap with each other will be referred to as an effective imaging range 21C below. A subject positioned in the effective imaging range 21C can be observed in a state where resolution is high and distortion does not appear.

In an auxiliary measurement light-emitting frame that emits planar auxiliary measurement light in the length measurement mode, the first auxiliary measurement light-emitting unit 30 emits planar auxiliary measurement light Lm in a state where a plane formed by planar auxiliary measurement light Lm crosses the optical axis Ax at a position P2. The auxiliary measurement light-emitting unit 30 allows the plane, which is formed by planar auxiliary measurement light Lm, to be within the effective imaging range 21C. The position P2 is present in the depth-of-field R1, and is a position where a distance L2 from the distal end portion of the objective lens 21 is in the range of 5 mm to 20 mm or 3 mm to 20 mm (a range particularly frequently used to observe a subject in endoscopy).

In a case where an object to be observed, such as a polyp, is present, a user, who uses the endoscope 12, operates the endoscope 12 so that the object to be observed is positioned in an optimum observation range for the object to be observed. Since an object to be observed is excessively big in the taken image in a case where the object to be observed is positioned on the near side of the optimum observation range, there is a case where it is not suitable for diagnosis. On the other hand, since it is difficult to observe the detailed state of an object to be observed in a case where the object to be observed is positioned on the back side of the optimum observation range, there is a case where it is not suitable for diagnosis. Accordingly, the object to be observed is frequently observed in a state where the object to be observed is positioned in the optimum observation range.

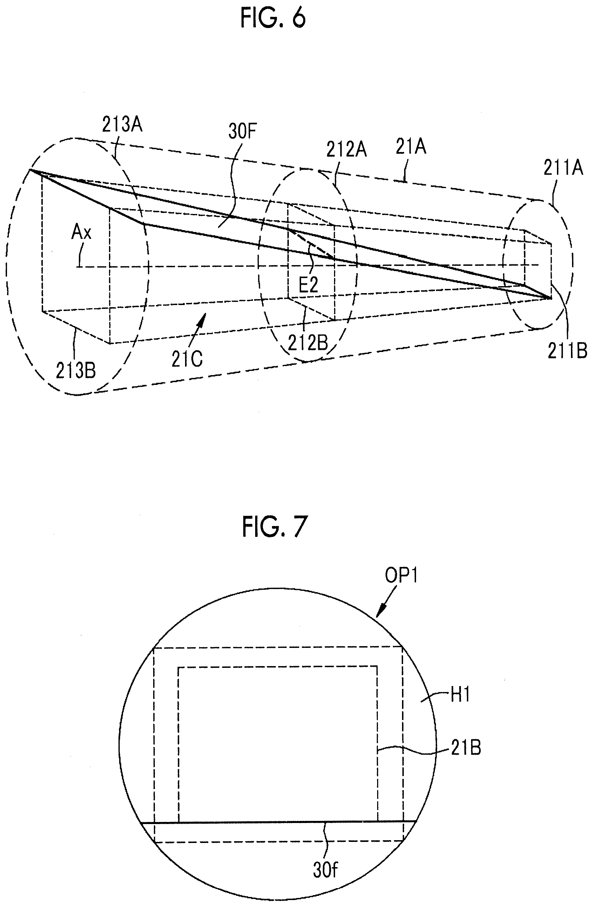

Fig. 6 shows a positional relationship between the visual field 21A and the effective imaging range 21C and a plane 30F, which is formed by planar auxiliary measurement light Lm. The visual field 21A has substantially the shape of a truncated cone, and the effective imaging range 21C has substantially the shape of a square column. A cross section 212A perpendicular to the optical axis Ax is present at the position P1 in the visual field 21A of the depth-of-field R1, and a cross section 211B perpendicular to the optical axis Ax is present at the position P1 in the effective imaging range 21C. Further, a cross section 212A perpendicular to the optical axis Ax is present at the position P2 in the visual field 21A of the depth-of-field R1, and a cross section 212B perpendicular to the optical axis Ax is present at the position P2 in the effective imaging range 21C. Furthermore, a cross section 213A perpendicular to the optical axis Ax is present at the position P3 in the visual field 21A of the depth-of-field R1, and a cross section 213B perpendicular to the optical axis Ax is present at the position P3 in the effective imaging range 21C.

The plane 30F, which is formed by planar auxiliary measurement light Lm, crosses the visual field 21A in a state where the plane 30F passes through an end portion of the cross section 211B of the effective imaging range 21C, passes through a center line E2 of the cross section 212B of the effective imaging range 21C, and passes through an end portion of the cross section 213B of the effective imaging range 21C. According to the above-mentioned structure, an optical image OP1 shown in Fig. 7 is obtained in a case where a planar subject H1 perpendicular to the optical axis Ax is disposed at the position P1 in the visual field 21A or the effective imaging range 21C when auxiliary measurement light is emitted.

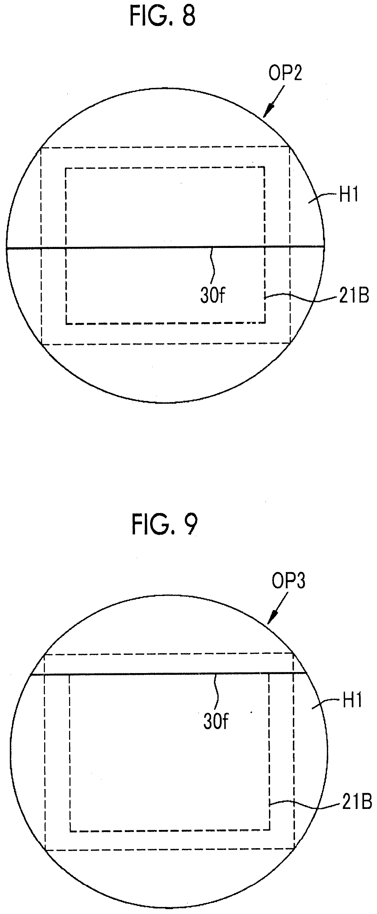

The crossing line 30f, which is formed between the subject H1 and the plane 30F in a case where the subject H1 is irradiated with planar auxiliary measurement light Lm, is displayed in the optical image OP1. The crossing line 30f is positioned on the lower side in the optical image OP1. The effective visual field 21B is subsidiarily shown in the optical image OP1 (the same applies to optical images OP2 and OP3 to be described later). Further, an optical image OP2 shown in Fig. 8 is obtained in a case where the subject H1 is disposed at the position P2 in the visual field 21A or the effective imaging range 21C when auxiliary measurement light is emitted. The crossing line 30f, which is formed between the subject H1 and the plane 30F, is also displayed in the optical image OP2 as in the optical image OP1. In a case where the optical image OP2 is compared with the optical image OP1, the position of the crossing line 30f in the optical image OP2 is higher than that in the optical image OP1.

Furthermore, an optical image OP3 shown in Fig. 9 is obtained in a case where the subject H1 is disposed at the position P3 in the visual field 21A or the effective imaging range 21C when auxiliary measurement light is emitted. The crossing line 30f, which is formed between the subject H1 and the plane 30F, is also displayed in the optical image OP3 as in the optical images OP1 and OP2. In a case where the optical image OP3 is compared with the optical image OP2, the position of the crossing line 30f in the optical image OP3 is higher than that in the optical image OP2.

The signal processing unit 39 generates a second taken image, which is used to set the gradations 70A, on the basis of the above-mentioned optical images OP1, OP2, and OP3. The generated second taken image is transmitted to the display control unit 40. The display control unit 40 acquires the second taken image obtained at the time of the auxiliary measurement light-emitting frame and a first taken image obtained at the time of an only-illumination light-emitting frame that emits only illumination light without emitting planar auxiliary measurement light. Then, the display control unit 40 sets a direction, in which the crossing line 30f included in the second taken image extends, as a horizontal direction. Accordingly, the extraction of the crossing line from the second taken image is completed. After that, the display control unit 40 displays the extracted crossing line 30f in the first taken image, so that a specific image including the crossing line 30f is displayed on the monitor 18.

In a case where the specific image is displayed on the monitor 18, the position of the crossing line 30f in the optical image in the vertical direction is changed due to a change in the distance to the subject on which the crossing line 30f is formed. That is, as the subject becomes distant from the objective lens 21, the crossing line 30f is moved up from the lower side of the display unit.

In a case where the display control unit 40 displays the specific image including the crossing line 30f on the monitor 18 on the basis of the optical image OP1 and the like, the display control unit 40 allows the crossing line 30f to overlap to display the gradations of the crossing line 30f that show an actual size. The gradations form gradations serving as the index of the size of a subject. A data table, which shows a relationship between a position in the vertical direction in the second taken image generated by the signal processing unit 39 and the actual size of one pixel of the image at the position, is stored in a ROM built in the display control unit 40.

A method of generating the data table is as follows. For example, a sheet of graph paper on which squares having a length of 1 mm and a width of 1 mm are arranged is prepared as the above-mentioned subject HI, and the graph paper is imaged by the imaging element 32 in a state where the graph paper is placed at an arbitrary distance from the distal end portion of the objective lens 21. Then, the position yn of the crossing line 30f of the second taken image in the vertical direction is obtained. Further, the length of the crossing line 30f included in the second taken image is measured using the squares of the graph paper. The measured length of the crossing line 30f is divided by the total number of pixels of the second taken image in the horizontal direction, so that the actual size of one pixel at the position is obtained. Then, information about the actual size of one pixel and the position yn are stored in the ROM in association with each other. The above-mentioned work is repeated while the position of the graph paper in the optical axis direction D3 is finely changed, so that the data table is made.

Specifically, the display control unit 40 detects the crossing line 30f from the second taken image generated by the signal processing unit 39, and uses one of a plurality of pixel data forming the crossing line 30f as a starting point. Then, the display control unit 40 sequentially selects pixel data from the starting point in the horizontal direction, and obtains information about the actual size of one pixel at the positions from the positions of the selected pixel data in the vertical direction and the data table.

The display control unit 40 integrates the actual size whenever the display control unit 40 selects pixel data, and specifies pixel data, which is selected in a case where an integrated value becomes the integer multiple of unit length (for example, 1 mm), as pixel data where gradations are to overlap. Further, the display control unit 40 also specifies the pixel data of the starting point as pixel data where gradations are to overlap. The display control unit 40 displays gradations (for example, vertical lines extending in the vertical direction), which show intervals corresponding to unit length, on the pixel data where gradations are to overlap. The gradations are displayed in the first taken image, so that a specific image including the gradations 70A (see Figs. 10 to 12), which serve as the index of the size of a subject, in addition to the crossing line 30f is displayed on the monitor 18.

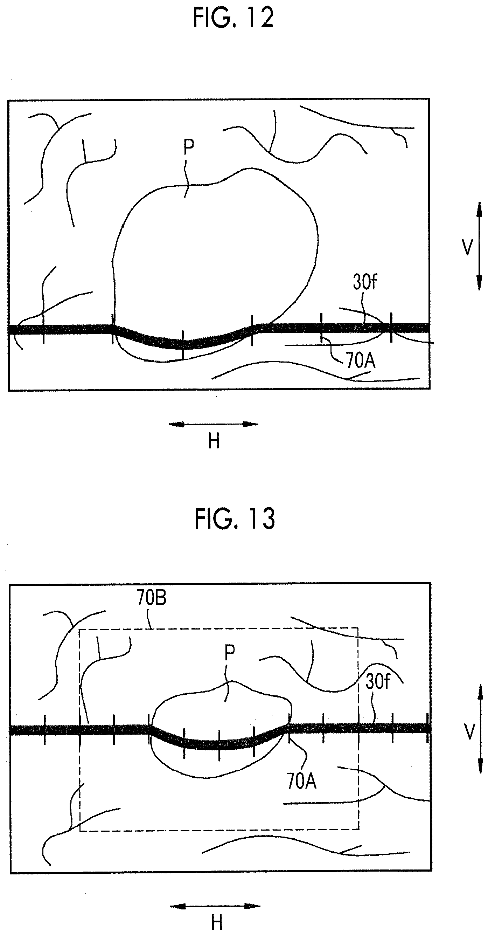

Fig. 10 shows a specific image in a case where a polyp P is present at a position corresponding to a distance L1 from the distal end portion of the objective lens 21, Fig. 11 shows a specific image in a case where a polyp P is present at a position corresponding to a distance larger than the distance L1, and Fig. 12 shows a specific image in a case where a polyp P is present at a position corresponding to a distance smaller than the distance L1. In Figs. 10 to 12, a direction H represents a horizontal direction in the display screen of the monitor 18 and a direction V represents a vertical direction of the display screen of the monitor 18. Further, the crossing line 30f and the gradations 70A showing unit length are displayed in the specific image displayed on the monitor 18 as shown in Figs. 10 to 12. A smaller interval between the gradations 70A is displayed as the crossing line 30f is closer to the upper side in the direction V in the display screen.

As described above, the length of an object to be measured can be obtained using the gradations 70A displayed in the specific image. For example, it is understood that the length of the polyp P in the direction H is about 4.5 mm as shown in Fig. 10 in a case where an interval between the gradations 70A is 1 mm.

As shown in Fig. 13, a frame 70B showing the effective visual field 21B may be displayed in the specific image displayed on the monitor 18. Since the frame 70B is displayed, it is possible to grasp whether or not a portion of the specific image is taken without distortion. For this reason, since the gradations 70A positioned outside the frame 70B are affected by distortion, it is possible to determine that the gradations 70A positioned outside the frame 70B should not be used for measurement. As a result, a measurement error is prevented.

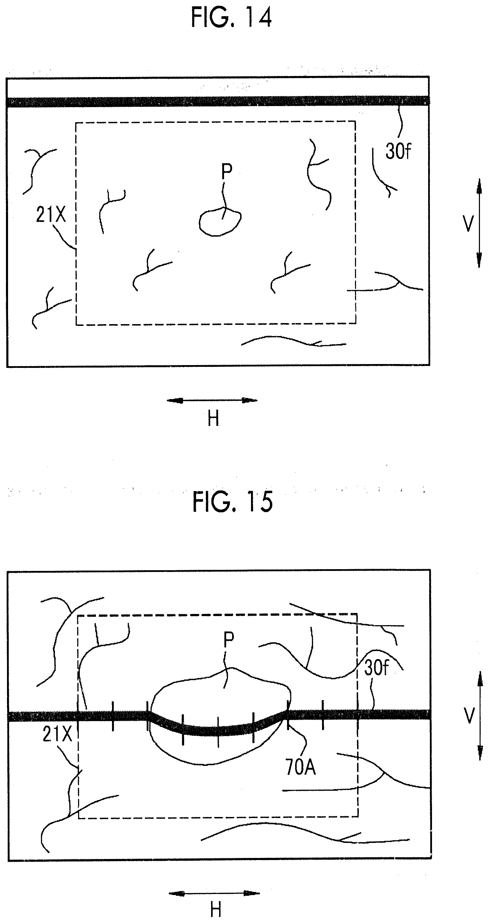

In a case where the crossing line 30f is positioned outside a range 21X corresponding to the effective visual field 21B in the specific image displayed on the monitor 18 as shown in Fig. 14, the gradations 70A are not displayed on the crossing line 30f. On the other hand, in a case where the crossing line 30f is positioned in the range 21X, the gradations 70A are displayed on the crossing line 30f. Accordingly, since it is possible to prevent measurement from being performed using the crossing line 30f, which is positioned in a range where distortion is large, a measurement error can be reduced. In the case where the crossing line 30f is positioned outside the range 21X, it is not necessary that gradations are not completely displayed and gradations may be displayed with a color different from the color of the gradations 70A or may be displayed with a line of which the type is different from the type of the line of the gradations 70A.

As shown in Fig. 15, in the specific image displayed on the monitor 18, the gradations 70A are displayed on only a portion of the crossing line 30f, which is positioned inside the range 21X corresponding to the effective visual field 21B, and are not displayed on a portion of the crossing line 30f that is positioned on the outside 21b of the range 21X. Since the gradations 70A are displayed on only a portion of the crossing line 30f overlapping with the effective visual field 21B as described above, the measurement of a portion having large distortion can be prevented. On the outside of the range 21X, instead of not displaying the gradations 70A, gradations may be displayed with a color different from the color of the gradations 70A or may be displayed with a line of which the type is different from the type of the line of the gradations 70A.

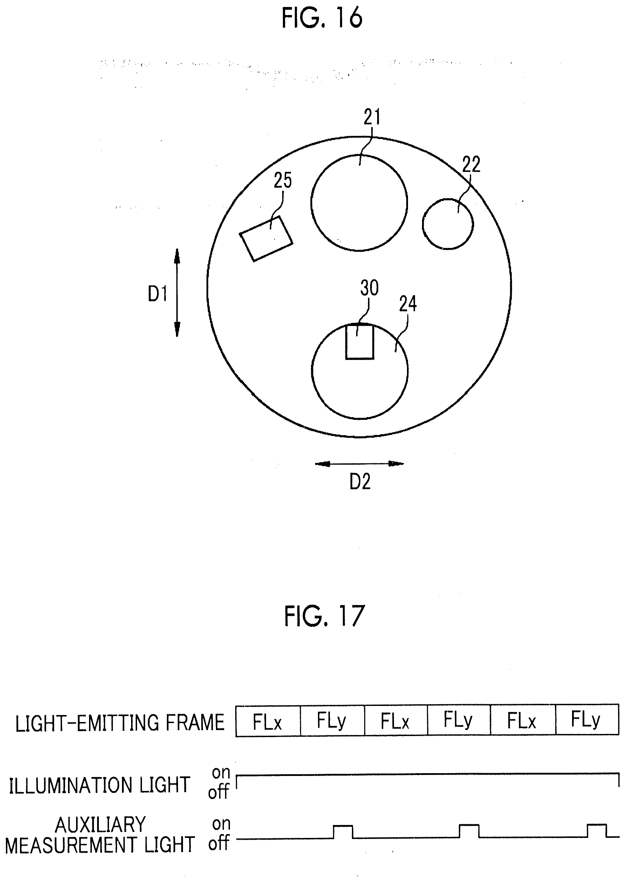

The first auxiliary measurement light-emitting unit 30 of the endoscope apparatus 10 may be adapted to be attachably and detachably mounted on the distal end portion 12d of the endoscope 12 other than a structure where the first auxiliary measurement light-emitting unit 30 of the endoscope apparatus 10 is fixed to the distal end portion 12d of the endoscope 12. In this case, the first auxiliary measurement light-emitting unit 30 may be adapted to be capable of being retrofitted to the opening 24 of the distal end portion 12d as an accessory as shown in Fig. 16.

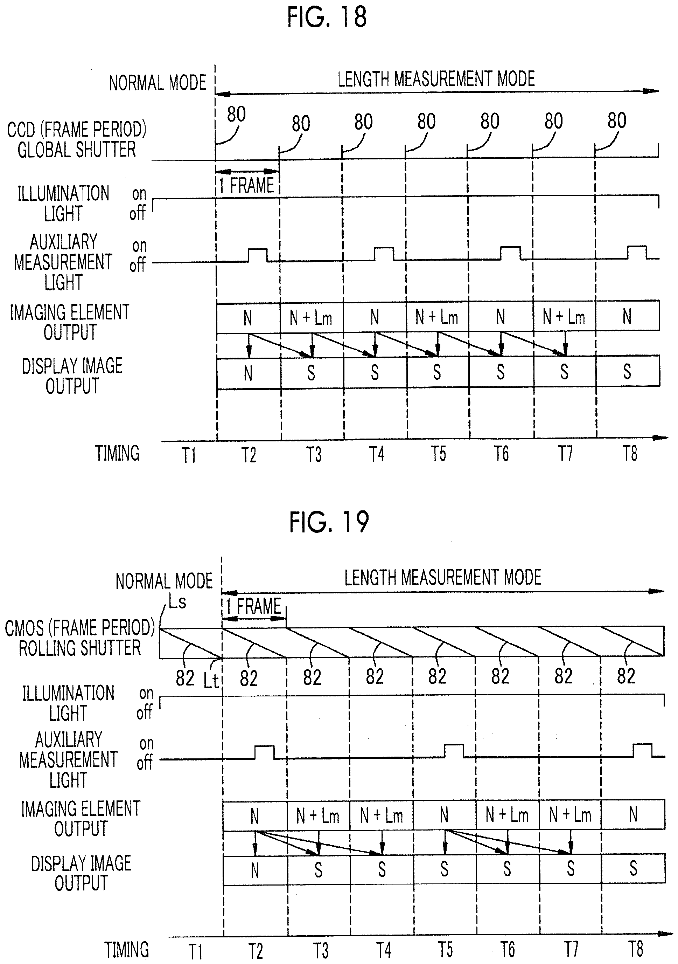

In the length measurement mode of this embodiment, the light source control unit 27 allows illumination light, which is used for the entire illumination of an object to be observed, to be continuously emitted and allows planar auxiliary measurement light Lm to be emitted in the form of a pulse. Accordingly, as shown in Fig. 17, an only-illumination light-emitting frame FLx that emits only illumination light without emitting planar auxiliary measurement light Lm and an auxiliary measurement light-emitting frame Fly that emits illumination light and planar auxiliary measurement light Lm are included as a frame that emits light in the length measurement mode. Then, in the length measurement mode, the extraction of the crossing line 30f from the second taken image obtained at the time of the auxiliary measurement light-emitting frame and the setting of the gradations 70A are performed and the crossing line 30f and the gradations 70A are displayed in the first taken image obtained at the time of the only-illumination light-emitting frame. Accordingly, a specific image, which includes the crossing line 30f and the gradations 70A, is displayed on the monitor 18. Therefore, since components of planar auxiliary measurement light are not included in the first taken image, the obstruction of the visibility of an object to be observed, which may be caused by the emission of planar auxiliary measurement light, does not occur. A solid line, which is shown in illumination light or planar auxiliary measurement light Lm of Fig. 17, shows the light-emitting state of a certain frame. A period where the solid line is positioned at a portion corresponding to "on" means a period where illumination light or planar auxiliary measurement light Lm is emitted, and a period where the solid line is positioned at a portion corresponding to "off' means a period where illumination light or planar auxiliary measurement light Lm is not emitted. The above-mentioned description of "on" and "off' is also applied to Figs. 18 to 22.

The patterns of light emission and imaging in the length measurement mode are as follows. A first pattern is a pattern in a case where a CCD (global shutter-type imaging element), which performs exposure and the reading of electric charges on the respective pixels at the same timing to output image signals, is used as the imaging element 32. Further, in the first pattern, planar auxiliary measurement light Lm is emitted at a two-frame interval as a specific frame interval.

In the first pattern, as shown in Fig. 18, the simultaneous reading of electric charges is performed (global shutter) on the basis of the exposure using illumination light at a timing T1 in the normal mode when the normal mode is switched to the length measurement mode (when the timing T1 is switched to a timing T2). As a result, a first taken image N including only components of illumination light is obtained. This first taken image N is displayed on the monitor 18 at the timing T2. In regard to "CCD (frame period) global shutter" of Fig. 18, a rising line 80 rising in the vertical direction means that global shutter is performed when the timing T1 is switched to the timing T2. The same applies to other rising lines 80.

Further, illumination light and auxiliary measurement light Lm are emitted at the timing T2. The simultaneous reading of electric charges is performed on the basis of the exposure using illumination light and planar auxiliary measurement light Lm, which is performed at the timing T2, when the timing T2 is switched to a timing T3. As a result, a second taken image N+Lm including components of illumination light and auxiliary measurement light Lm is obtained. The extraction of the crossing line 30f and the setting of the gradations 70A are performed on the basis of this second taken image N+Lm. The crossing line 30f and the gradations 70A are displayed in the first taken image N that is displayed at the timing T2. Accordingly, a specific image S where the crossing line 30f and the gradations 70A are displayed in the first taken image N displayed at the timing T2 is displayed at the timing T3.

The first taken image N displayed at the timing T2 (first timing) is displayed on the monitor 18 not only at the timing T2 but also at the timing T3. That is, the first taken image displayed at the timing T2 is continuously displayed over two frames until a timing T4 (second timing) when the next first taken image is obtained (the same subject image is displayed at the timings T2 and T3). The second taken image N+Lm is not displayed on the monitor 18 at the timing T3. Here, in the normal mode, the first taken image N is displayed while being changed for each frame. However, since the same first taken image N2 is continuously displayed over two frames as described above in the first pattern of the length measurement mode, a frame rate in the first pattern of the length measurement mode is substantially 1/2 of that in the normal mode.

An image is displayed even at a timing T4 or later in the same way. That is, the first taken image displayed at the timing T4 is continuously displayed in the specific image S at the timings T4 and T5, and a first taken image N displayed at the timing T6 is continuously displayed in the specific image S at the timings T6 and T7. In contrast, a second taken image N+Lm is not displayed on the monitor 18 at the timings T4, T5, T6, and T7. Since the first taken image N, which does not include the components of planar auxiliary measurement light, is displayed for the display of the specific image S as described above, a frame rate is slightly reduced but the obstruction of the visibility of an object to be observed, which may be caused by the emission of planar auxiliary measurement light Lm, does not occur.

A second pattern is a pattern in a case where a CMOS (rolling shutter-type imaging element), which includes a plurality of lines used to image an object to be observed illuminated with illumination light or planar auxiliary measurement light Lm, performs exposure at different exposure timings for the respective lines, and reads electric charges at different reading timings for the respective lines to output image signals, is used as the imaging element 32. Further, in the second pattern, planar auxiliary measurement light Lm is emitted at a three-frame interval as a specific frame interval.

In the second pattern, as shown in Fig. 19, the exposure using illumination light and the reading of electric charges are performed for each line at the timing T1 and the reading of electric charges is completed (rolling shutter) when the normal mode is switched to the length measurement mode (when the timing T1 is switched to the timing T2).

As a result, a first taken image N including only components of illumination light is obtained. This first taken image N is displayed on the monitor 18 at the timing T2. In regard to "CMOS (frame period) rolling shutter" of Fig. 19, a diagonal line 82 means a timing when the exposure using light and the reading of the electric charge are performed, a line Ls means that the exposure and the reading of electric charges are started, and a line Lt means that the exposure and the reading of electric charges are completed. The same applies to other diagonal lines 82, and applies to third to fifth patterns.

Further, illumination light and auxiliary measurement light Lm are emitted at the timing T2. Rolling shutter is performed on the basis of illumination, which is performed using illumination light to the timing T2 from the timing T1, and illumination that is performed using planar auxiliary measurement light Lm at the timing T2. Accordingly, a second taken image N+Lm including components of illumination light and planar auxiliary measurement light Lm is obtained when the timing T2 is switched to the timing T3. Furthermore, even when the timing T3 is switched to the timing T4, a second taken image N+Lm including the components of illumination light and planar auxiliary measurement light Lm is obtained. The extraction of the crossing line 30f and the setting of the gradations 70A are performed on the basis of the second taken image N+Lm. Planar auxiliary measurement light Lm is not emitted at the timings T3 and T4.

The crossing line 30f and the gradations 70A are displayed in the first taken image N that is displayed at the timing T2. Accordingly, a specific image S where the crossing line 30f and the gradations 70A are displayed in the first taken image N displayed at the timing T2 is displayed at the timings T3 and T4. The first taken image N displayed at the timing T2 (first timing) is displayed on the monitor 18 not only at the timing T2 but also at the timings T3 and T4. That is, the first taken image displayed at the timing T2 is continuously displayed over three frames until a timing T5 (second timing) when the next first taken image is obtained (the same subject image is displayed at the timings T2, T3, and T4). In contrast, the second taken image N+Lm is not displayed on the monitor 18 at the timings T3 and T4. Since the same first taken image N2 is continuously displayed over three frames in the second pattern of the length measurement mode, a frame rate in the second pattern of the length measurement mode is substantially 1/3 of that in the normal mode.

An image is displayed even at a timing T5 or later in the same way. The first taken image displayed at the timing T5 is displayed in the specific image S at the timings T5, T6, and T7. In contrast, a second taken image N+Lm is not displayed on the monitor 18 at the timings T5, T6, and T7. Since the first taken image, which does not include the components of planar auxiliary measurement light, is displayed for the display of the specific image S as described above, a frame rate is reduced but the obstruction of the visibility of an object to be observed, which may be caused by the emission of planar auxiliary measurement light Lm, does not occur.

A third pattern is a pattern in a case where a rolling shutter-type imaging element is used as the imaging element 32 as in the second pattern. Further, in the third pattern, planar auxiliary measurement light Lm is emitted at a two-frame interval as a specific frame interval.

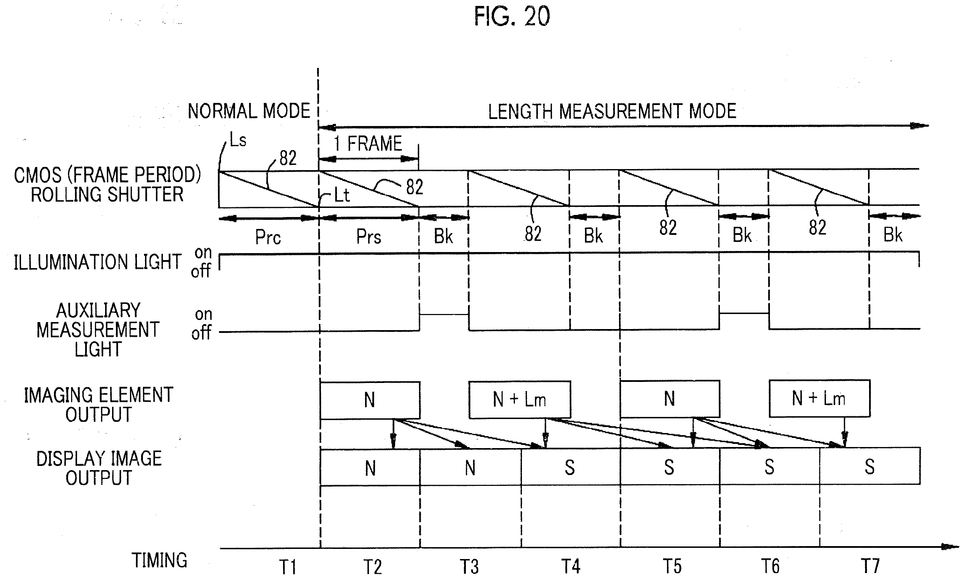

In the third pattern, as shown in Fig. 20, the exposure using illumination light and the reading of electric charges are performed for each line at the timing T1 and the reading of electric charges is completed (rolling shutter) when the normal mode is switched to the length measurement mode (when the timing T1 is switched to the timing T2). As a result, a first taken image N including only components of illumination light is obtained. This first taken image N is displayed on the monitor 18 during the timings T2 and T3 after the normal mode is switched to the length measurement mode. The reading period Prc of the imaging element 32 in the normal mode and the reading period Prs of the imaging element 32 in the third pattern of the length measurement mode are set equal to each other. Accordingly, an imaging frame rate is lower than a display frame rate (the display of an image to the timing T4 from the timing T2 corresponds to three frames, but the output of a taken image corresponds to two frames).

Further, a blanking period Bk in which the output of image signals to be performed by the reading of electric charges is prohibited is provided between the respective reading periods (until the start of reading of electric charges after the completion of reading of electric charges) in the third pattern. Planar auxiliary measurement light Lm is emitted in this blanking period Bk. Rolling shutter is performed on the basis of illumination using illumination light and planar auxiliary measurement light Lm, so that a second taken image N+Lm including components illumination light and planar auxiliary measurement light Lm is obtained. The extraction of the crossing line 30f and the setting of the gradations 70A are performed on the basis of the second taken image N+Lm. The crossing line 30f and the gradations 70A are displayed in the first taken image N that is displayed at the timing T2. Then, a specific image S where the crossing line 30f and the gradations 70A are displayed in the first taken image N displayed at the timing T2 is displayed at the timing T4.

An image is displayed even at a timing T5 or later in the same way. The first taken image N displayed at the timing T5 is displayed in the specific image S at a timing T5 (first timing), a timing T6, and a timing T7 until a first taken image N is obtained at the next timing T8 (second timing). That is, the first taken image N displayed at the timing T5 is continuously displayed at the timings T5, T6, and T7 (the same subject image is displayed at the timings T5, T6, and T7). In contrast, the second taken image N+Lm is not displayed at the timings T5, T6, and T7. Since the first taken image, which does not include the components of planar auxiliary measurement light, is displayed for the display of the specific image S as described above, a frame rate is reduced but the obstruction of the visibility of an object to be observed, which may be caused by the emission of planar auxiliary measurement light, does not occur. Since the same first taken image N2 is continuously displayed over three frames in the third pattern of the length measurement mode, a frame rate in the third pattern of the length measurement mode is substantially 1/3 of that in the normal mode.

The crossing line 30f and the gradations 70A, which are extracted and set from the second taken image N obtained before the timing T5 are used in the specific image S displayed at the timings T5 and T6, and the crossing line 30f and the gradations 70A, which are extracted and set from the second taken image obtained after the timing T5 are used in the specific image S displayed at the timing T7.

A fourth pattern is a pattern in a case where a rolling shutter-type imaging element is used as the imaging element 32 as in the second pattern. Further, in the fourth pattern, planar auxiliary measurement light Lm is emitted at a two-frame interval as a specific frame interval.

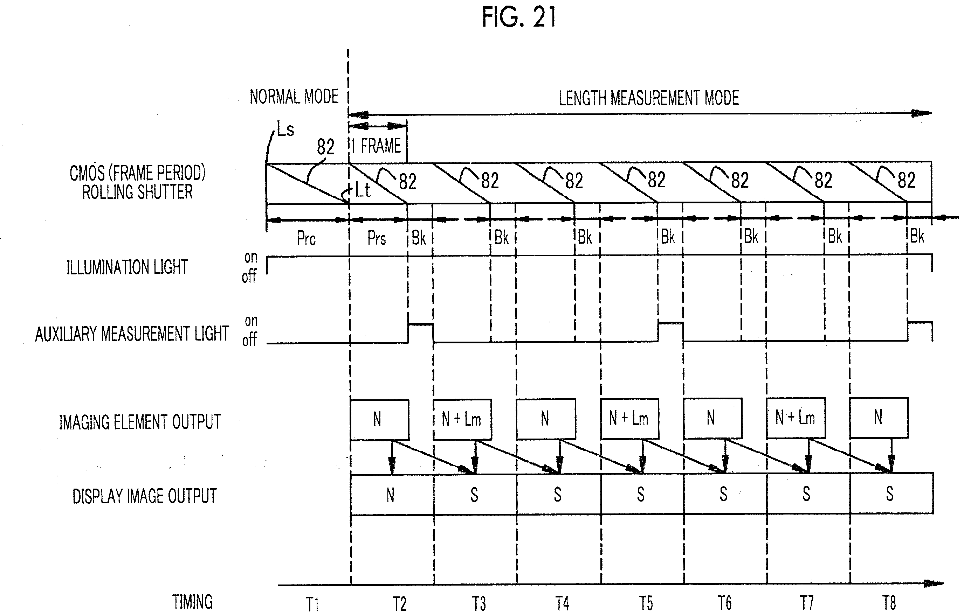

In the fourth pattern, as shown in Fig. 21, the exposure using illumination light and the reading of electric charges are performed for each line at the timing T1 and the reading of electric charges is completed (rolling shutter) when the normal mode is switched to the length measurement mode (when the timing T1 is switched to the timing T2). As a result, a first taken image N including only components of illumination light is obtained. This first taken image N is displayed on the monitor 18 at the timing T2. To make an imaging frame rate and a display frame rate equal to each other, the reading period Prs of the imaging element 32 in the fourth pattern of the length measurement mode is set shorter than the reading period Prc of the imaging element 32 in the normal mode as shown in the following equation 1).

$$\text{Equation 1) } Prc(=\text{display frame rate})=Prs+Bk$$

Here, Prc is 1/60 in a case where the display frame rate is set to 1/60 sec. In this case, it is preferable that, for example, Prs is set to 1/90 sec and Bk is set to 1/180 sec.

Further, a blanking period Bk in which the output of image signals to be performed by the reading of electric charges is prohibited is provided between the respective reading periods in the fourth pattern. Planar auxiliary measurement light Lm is emitted in this blanking period Bk. Rolling shutter is performed on the basis of illumination using illumination light and planar auxiliary measurement light Lm in the blanking period Bk, so that a second taken image N+Lm including components illumination light and planar auxiliary measurement light is obtained. The extraction of the crossing line 30f and the setting of the gradations 70A are performed on the basis of the second taken image N+Lm. The crossing line 30f and the gradations 70A are displayed in the first taken image N that is displayed at the timing T2. Then, a specific image S where the crossing line 30f and the gradations 70A are displayed in the first taken image N displayed at the timing T2 is displayed at the timing T3. That is, the first taken image N displayed at the timing T2 is continuously displayed over two frames (the same subject image is displayed at the timings T2 and T3). On the other hand, the second taken image N+Lm is not displayed at the timing T3.

An image is displayed even at the timing T4 or later in the same way. The first taken image displayed at the timing T4 is used in the specific image S at the timings T4 and T5. Further, the first taken image displayed at the timing T6 is used in the specific image S at the timings T6 and T7. On the other hand, a second taken image N+Lm is not displayed at the timings T4, T5, T6, and T7. Since the first taken image, which does not include the components of planar auxiliary measurement light, is used for the display of the specific image S as described above, a frame rate is reduced but the obstruction of the visibility of an object to be observed, which may be caused by the emission of planar auxiliary measurement light, does not occur.

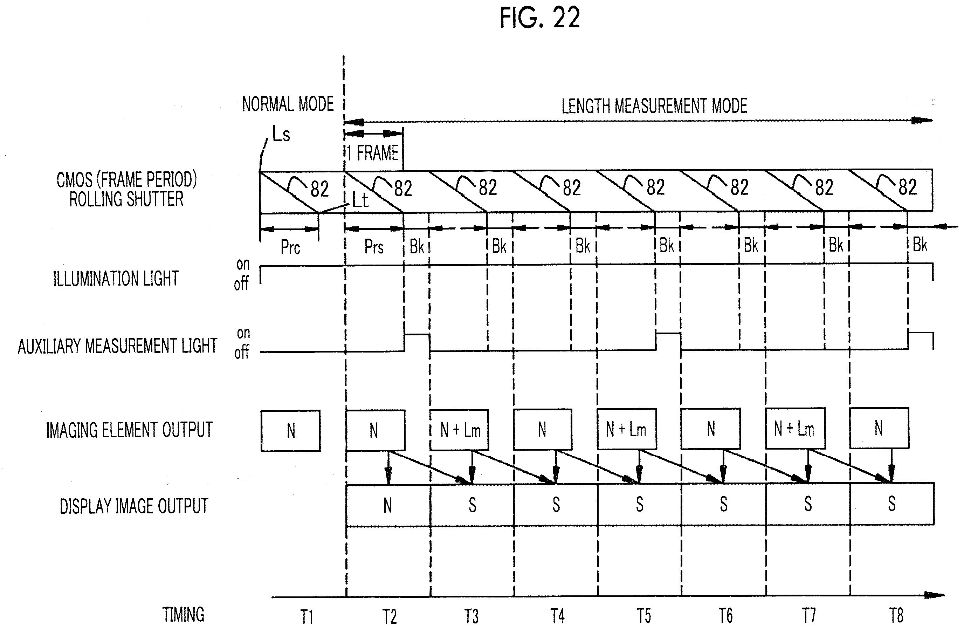

A fifth pattern is the same as the fourth pattern except that a reading period in the normal mode is set equal to a reading period in the length measurement mode. That is, as shown in Fig. 22, a rolling shutter-type imaging element is used in the fifth pattern as the imaging element 32 as in the second pattern. Further, in the fifth pattern, planar auxiliary measurement light Lm is emitted at a two-frame interval as a specific frame interval. Furthermore, a blanking period Bk is provided and planar auxiliary measurement light Lm is made to be emitted in the blanking period Bk. Rolling shutter is performed on the basis of illumination performed using illumination light and planar auxiliary measurement light Lm in the blanking period Bk, so that a second taken image N+Lm including components of illumination light and planar auxiliary measurement light is obtained. Then, the extraction of the crossing line 30f and the setting of the gradations 70A are performed on the basis of the second taken image N+Lm.

The crossing line 30f and the gradations 70A are displayed in the first taken image N that is displayed at the timing T2. Then, a specific image S where the crossing line 30f and the gradations 70A are displayed in the first taken image N displayed at the timing T2 is displayed at the timing T3. That is, the first taken image N displayed at the timing T2 is continuously displayed over two frames (the same subject image is displayed at the timings T2 and T3). On the other hand, the second taken image N+Lm is not displayed at the timing T3. Since the first taken image, which does not include the components of planar auxiliary measurement light, is used for the display of the specific image S as described above, a frame rate is reduced but the obstruction of the visibility of an object to be observed, which may be caused by the emission of planar auxiliary measurement light Lm, does not occur. Further, since a reading period in the normal mode is equal to a reading period in the length measurement mode and the same blanking period is provided in each of the respective modes (that is, a method of driving the imaging element 32 is not changed in the respective modes) in the fifth pattern, a mode is smoothly switched without the disturbance of the image (interruption, blackout, or stop) in a case where the normal mode is to be switched to the length measurement mode.

The reading period Prs of the imaging element 32 in the fifth pattern of the length measurement mode is set shorter than the reading period Prc of the imaging element 32 in the normal mode as shown in the following equation 2).

$$\text{Equation 2) } Prc+Bk=Prs+Bk(=display\ frame\ rate)$$

Here, it is preferable that, for example, Prc and Prs are set to 1/90 sec and Bk is set to 1/180 sec in a case where the display frame rate is set to 1/60 sec.

Second embodiment

According to a second embodiment, which is disclosed for exemplary purpose only and does not form part of the claimed invention, in a length measurement mode, spot-like auxiliary measurement light is used instead of planar auxiliary measurement light Lm and a substantially circular area (spot) is formed on a subject by spot-like auxiliary measurement light. Then, a second measurement marker, which represents the actual size of the subject, is generated on the basis of the position of this spot and is displayed on a first taken image. A light-emitting frame of the length measurement mode is the same as that in the first embodiment in that an only-illumination light-emitting frame FLx emitting only illumination light without emitting spot-like auxiliary measurement light and an auxiliary measurement light-emitting frame Fly emitting illumination light and spot-like auxiliary measurement light are emitted at specific intervals (see Fig. 17).

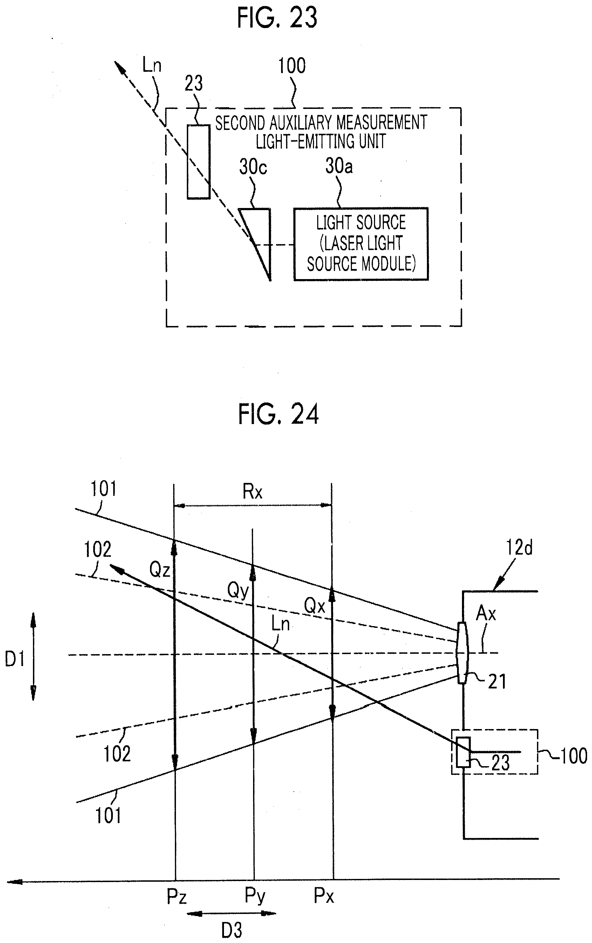

As shown in Fig. 23, a second auxiliary measurement light-emitting unit 100 does not include the DOE 30b, which is used to make light planar, unlike the first auxiliary measurement light-emitting unit 30. Further, it is preferable that a laser light source module is used as a light source 30a of the second auxiliary measurement light-emitting unit 100 to emit spot-like auxiliary measurement light. It is preferable that the laser light source module is a pigtail-type module (TOSA; Transmitter Optical Sub Assembly) comprising a visible laser diode (VLD) emitting laser light in a visible wavelength range and a condenser lens condensing laser light emitted from the VLD.

A prism 30c is an optical member that is used to change the travel direction of spot-like auxiliary measurement light emitted from the light source unit. The prism 30c changes the travel direction of spot-like auxiliary measurement light so that spot-like auxiliary measurement light crosses the visual field of an imaging optical system including an objective lens 21 and lens groups. A subject is irradiated with spot-like auxiliary measurement light, which is emitted from the prism 30c, through an auxiliary measurement lens 23.

In an auxiliary measurement light-emitting frame that emits auxiliary measurement light in the length measurement mode, the second auxiliary measurement light-emitting unit 100 emits spot-like auxiliary measurement light in a state where an optical axis Ln of the spot-like auxiliary measurement light crosses an optical axis Ax of the objective lens 21 as shown in Fig. 24. In a case where a subject can be observed in a range Rx of an observation distance, it is understood that the positions (points where the respective arrows Qx, Qy, and Qz cross the optical axis Ax) of the substantially circular areas (hereinafter, referred to as spots) formed on the subject by spot-like auxiliary measurement light in imaging ranges (shown by arrows Qx, Qy, and Qz) at a near end Px, an intermediate vicinity Py, and a far end Pz of the range Rx are different from each other. The imaging angle of view of the imaging optical system is represented by an area between two solid lines 101, and measurement is performed in a central area (an area between two dotted lines 102), in which an aberration is small, of this imaging angle of view.

Since spot-like auxiliary measurement light is emitted in the second embodiment in a state where the optical axis Ln of spot-like auxiliary measurement light crosses the optical axis Ax as described above, sensitivity to the movement of the position of a spot with respect to a change in the observation distance is high. Accordingly, the size of the subject can be measured with high accuracy. Then, the subject illuminated with spot-like auxiliary measurement light is imaged by the imaging element 32, so that a second taken image including a spot is obtained. In the second taken image, the position of a spot depends on a relationship between the optical axis Ax of the objective lens 21 and the optical axis Ln of auxiliary measurement light and an observation distance. The number of pixels showing the same actual size (for example, 5 mm) is increased in the case of a short observation distance, and the number of pixels showing the same actual size (for example, 5 mm) is reduced in the case of a long observation distance.

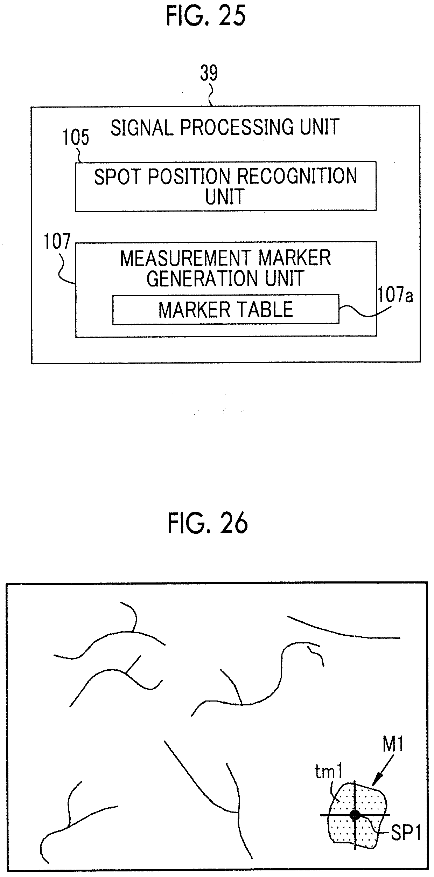

Accordingly, in a case where information showing a relationship between the position of a spot and the size (the number of pixels) of the second measurement marker corresponding to the actual size of a subject is stored in advance as described in detail below, the size of the measurement marker can be calculated from the position of the spot. In the second embodiment, a signal processing unit 39 of a processor device 16 includes a spot position recognition unit 105 and a measurement marker generation unit 107 as shown in Fig. 25 so as to perform the recognition of the position of a spot, the calculation of the size of the second measurement marker, and the generation of the second measurement marker.

It is preferable that the spot position recognition unit 105 recognizes the position of a spot from an image, which includes many components corresponding to the color of auxiliary measurement light, of the second taken image. Since auxiliary measurement light includes, for example, many red components, it is preferable that the spot position recognition unit 105 recognizes the position of a spot from a red image of the second taken image. As a method of recognizing the position of a spot, for example, there is a method including binarizing a red image of the second taken image and recognizing the center of a white portion (a pixel where signal strength is higher than a threshold value for binarization) of the binarized image as the position of a spot.

The measurement marker generation unit 107 generates a second measurement marker, which represents the actual size of a subject, on the basis of the position of the spot in the second taken image. The measurement marker generation unit 107 calculates the size of a marker from the position of the spot with reference to a marker table 107a where a relationship between the position of a spot in the second taken image and a second measurement marker representing the actual size of a subject is stored. Then, the measurement marker generation unit 107 generates a second measurement marker corresponding to the size of the marker.

After the recognition of the position of the spot and the generation of the second measurement marker are completed, a display control unit 40 displays a spot display portion and the second measurement marker at the position of the spot in a first taken image (where the spot does not appear), which is obtained through the imaging of the subject illuminated with illumination light, on a monitor 18. For example, a cruciform measurement marker is used as the second measurement marker in the second embodiment. As shown in Fig. 26, a cruciform marker M1, which represents the actual size of 5 mm (a horizontal direction and a vertical direction of the second taken image), is displayed at the center of a spot display portion SP1 formed on a tumor tm1 of a subject in a case where an observation distance is close to the near end Px. Since the tumor tm1 and a range, which is determined by the cruciform marker M1, substantially match each other, the size of the tumor tm1 can be measured as about 5 mm. In the first taken image, the spot display portion may not be displayed and only the second measurement marker may be displayed.

Since a spot formed by auxiliary measurement light does not appear in the first taken image, a spot display portion is displayed at a portion, which corresponds to the position of the recognized spot, with brightness and a color that allow a user to know the position of the spot. In a case where the spot and a portion of the subject to be observed have the same color (red color), the visibility of the portion to be observed may deteriorate due to the spread of the color. However, since a spot display portion representing the spot is displayed in the first taken image where the spot does not appear as described above, the spread of the color caused by auxiliary measurement light can be avoided. Accordingly, the visibility of the portion to be observed does not deteriorate.

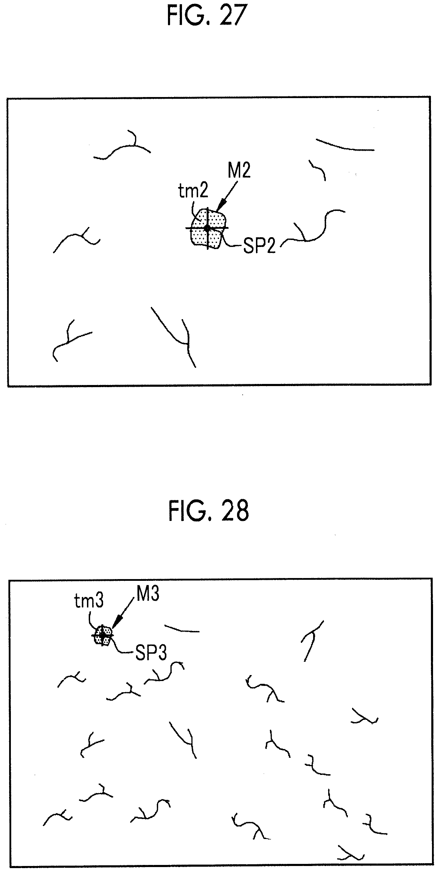

Similarly, as shown in Fig. 27, a cruciform marker M2, which represents the actual size of 5 mm (a horizontal direction and a vertical direction of the second taken image), is displayed at the center of a spot display portion SP2 formed on a tumor tm2 of a subject in a case where an observation distance is close to the intermediate vicinity Py. Further, as shown in Fig. 28, a cruciform marker M3, which represents the actual size of 5 mm (a horizontal direction and a vertical direction of the second taken image), is displayed at the center of a spot display portion SP3 formed on a tumor tm3 of a subject. As described above, the position of a spot on the imaging surface of the imaging element 32 varies according to an observation distance. For this reason, the display position of the marker also varies. As shown in Figs. 26 to 28, the size of the second measurement marker corresponding to the same actual size of 5 mm is reduced as an observation distance is increased.

The spot and the marker are displayed in Figs. 26 to 28 so that the center of the spot and the center of the marker match each other, but the second measurement marker may be displayed at a position apart from the spot in a case where a problem in terms of measurement accuracy does not occur. Even in this case, it is preferable that the second measurement marker is displayed near the spot. Further, a deformed second measurement marker is not displayed, and the distortion of a taken image may be corrected and an undeformed second measurement marker may be displayed in a corrected taken image.

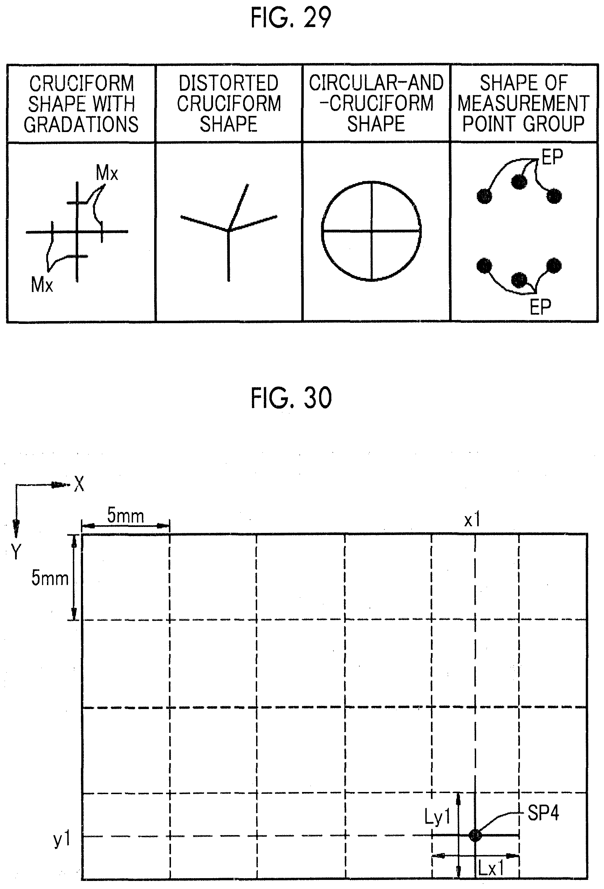

Further, the second measurement marker corresponding to the actual size of a subject of 5 mm is displayed in Figs. 26 to 28, but the actual size of a subject may be set to any value (for example, 2 mm, 3 mm, 10 mm, or the like) according to an object to be observed or the purpose of observation. Furthermore, in Figs. 26 to 28, the second measurement marker has a cruciform shape where a vertical line and a horizontal line are orthogonal to each other. However, as shown in Fig. 29, the second measurement marker may have a cruciform shape with gradations where gradations Mx are given to at least one of a vertical line or a horizontal line of a cruciform shape. Further, the second measurement marker may have a distorted cruciform shape of which at least one of a vertical line or a horizontal line is inclined. Furthermore, the second measurement marker may have a circular-and-cruciform shape where a cruciform shape and a circle are combined with each other. In addition, the second measurement marker may have the shape of a measurement point group where a plurality of measurement points EP corresponding to an actual size from a spot display portion are combined with each other. Further, one second measurement marker may be displayed or a plurality of second measurement markers may be displayed, and the color of the second measurement marker may be changed according to an actual size.

A method of making the marker table 107a will be described below. A relationship between the position of a spot and the size of a marker can be obtained through the imaging of a chart where a pattern having the actual size is regularly formed. For example, spot-like auxiliary measurement light is emitted to the chart; a graph paper-shaped chart including lines (5 mm) having the same size as the actual size or lines (for example, 1 mm) having a size smaller than the actual size is imaged while an observation distance is changed to change the position of a spot; and a relationship between the position of a spot (pixel coordinates of the spot on the imaging surface of the imaging element 32) and the number of pixels corresponding to the actual size (pixels showing 5 mm that is the actual size) is acquired.

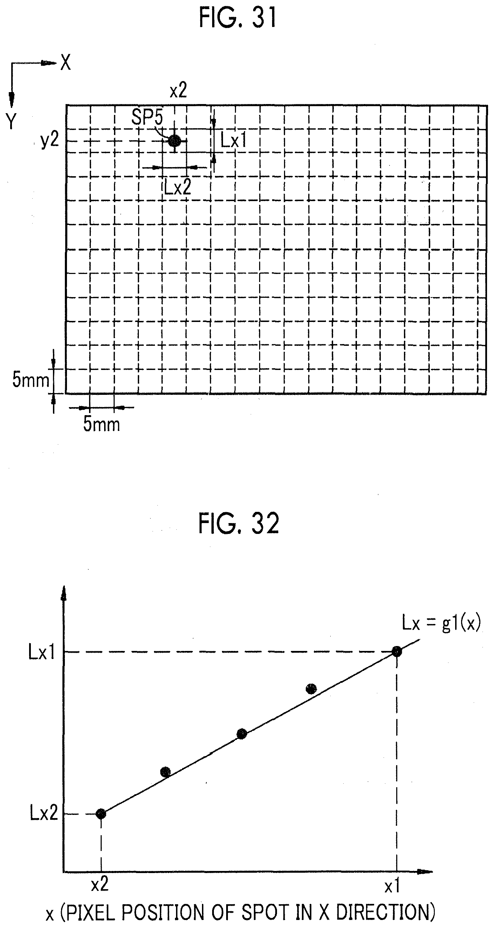

As shown in Fig. 30, (x1,y1) means the pixel position of a spot SP4 in an X direction and a Y direction on the imaging surface of the imaging element 32 (an upper left point is the origin of a coordinate system). The number of pixels in the X direction, which corresponds to the actual size of 5 mm, at the position (x1,y1) of the spot SP4 is denoted by Lx1, and the number of pixels in the Y direction is denoted by Ly1. This measurement is repeated while an observation distance is changed. Fig. 31 shows a state where the chart including lines having a size of 5 mm as in Fig. 30 is imaged, but an interval between the lines is narrow since this state is a state where an observation distance is closer to the far end than that in the state of Fig. 30. In the state of Fig. 31, the number of pixels in the X direction, which corresponds to the actual size of 5 mm, at the position (x2,y2) of a spot SP5 on the imaging surface of the imaging element 32 is denoted by Lx2, and the number of pixels in the Y direction is denoted by Ly2. Then, while an observation distance is changed, the same measurement as those in Figs. 30 and 31 is repeated and the results thereof are plotted. The charts are shown in Fig. 30 and 31 without consideration for the distortion of the objective lens 21.

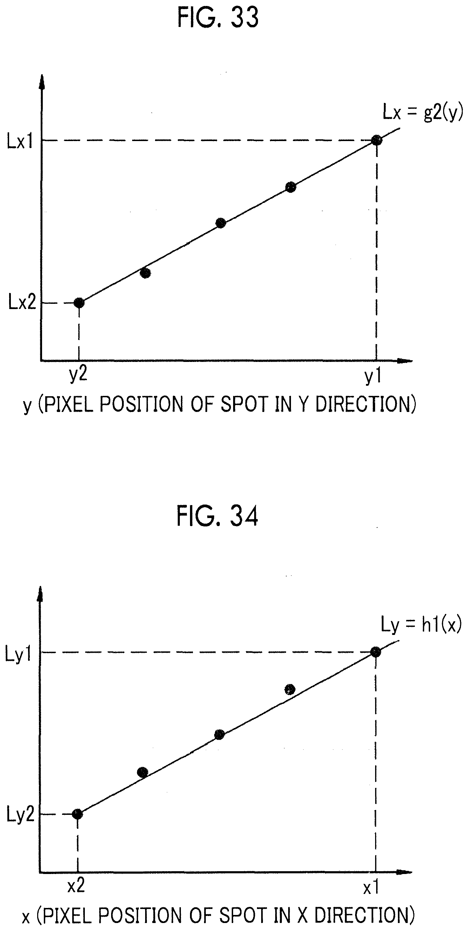

Fig. 32 shows a relationship between the X-coordinate of the position of a spot and Lx (the number of pixels of the second measurement marker in the X direction), and Fig. 33 shows a relationship between the Y-coordinate of the position of a spot and Lx. Lx is expressed by "Lx=g1(x)" as a function of the position in the X direction from the relationship of Fig. 32, and Lx is expressed by "Lx=g2(y)" as a function of the position in the Y direction from the relationship of Fig. 33. The functions g1 and g2 are obtained from the above-mentioned plotted results by, for example, a least-square method.

The X-coordinate of a spot corresponds to the Y-coordinate of a spot one to one, and basically the same results are obtained (the same number of pixels is obtained at the position of the same spot) even though any one of the function g1 or g2 is used. Accordingly, in a case where the size of the second measurement marker is to be calculated, any one of the function g1 or g2 may be used and a function of which sensitivity to a change in the number of pixels with respect to a change in position is higher may be selected from the functions g1 and g2. Further, in a case where the values of the functions g1 and g2 are significantly different from each other, it may be determined that "the position of a spot cannot be recognized".

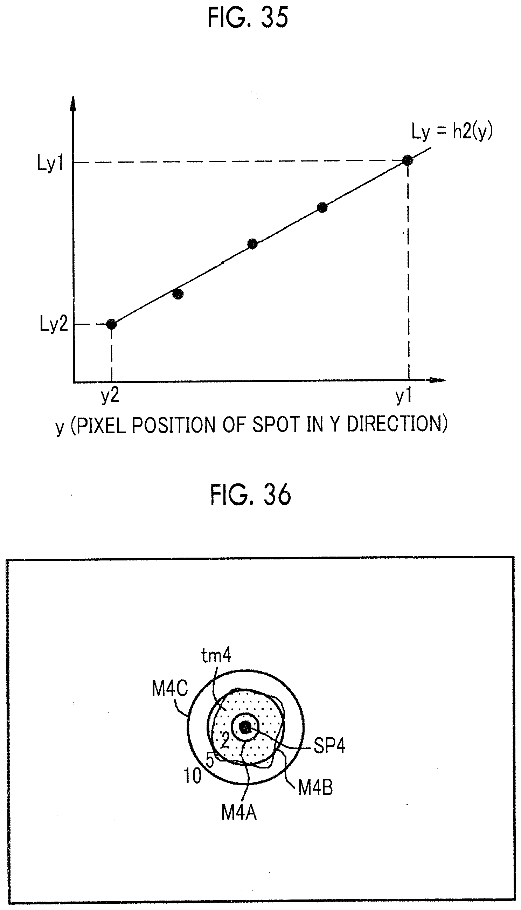

Fig. 34 shows a relationship between the X-coordinate of the position of a spot and Ly (the number of pixels in the Y direction), and Fig. 35 shows a relationship between the Y-coordinate of the position of a spot and Ly. Ly is expressed by "Ly=hl(x)" as the coordinate of the position in the X direction from the relationship of Fig. 34, and Ly is expressed by "Ly=h2(y)" as the coordinate of the position in the Y direction from the relationship of Fig. 35. Any one of the function h1 or h2 may also be used as Ly as in the case of Lx.

The functions g1, g2, h1, and h2 obtained as described above are stored in a marker table in the form of a look-up table. The functions g1 and g2 may be stored in a marker table in the form of a function.

In the second embodiment, as shown in Fig. 36, three concentric circular markers M4A, M4B, and M4C having different sizes (of which diameters are 2 mm, 5 mm, and 10 mm, respectively) may be displayed around a spot display portion SP4 formed on a tumor tm4 in the first taken image as the second measurement marker. In the case of the three concentric circular markers, it is possible to save a trouble of switching a marker since the plurality of markers are displayed, and it is possible to perform measurement even in a case where a subject has a non-linear shape. In a case where a plurality of concentric circular markers are displayed around a spot, a size and a color are not designated for each marker but combinations of a plurality of conditions may be prepared in advance and one can be selected from these combinations.

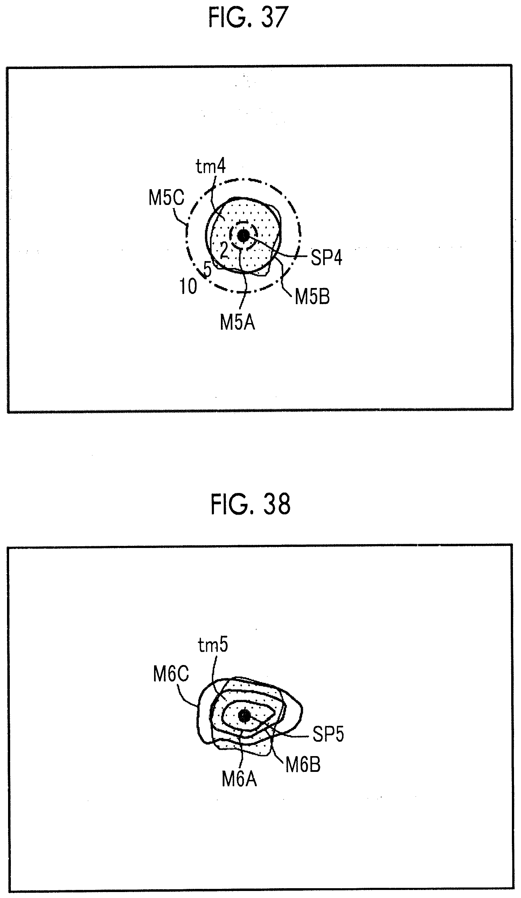

In Fig. 36, all the three concentric circular markers are displayed with the same color (black). However, in a case where a plurality of concentric circular markers are to be displayed, a plurality of color concentric circular markers of which colors are different from each other may be used. As shown in Fig. 37, a marker M5A is displayed by a dotted line representing a red color, a marker M5B is displayed by a solid line representing a blue color, and a marker M5C is displayed by a one-dot chain line representing a white color. Since identifiability can be improved in a case where the colors of the markers are changed in this way, measurement can be easily performed.

Further, as shown in Fig. 38, a plurality of distorted concentric circular markers, which are distorted from the respective concentric circles, may be used as the second measurement marker other than the plurality of concentric circular markers. In this case, distorted concentric circular markers M6A, M6B, and M6C are displayed around a spot display portion SP5, which is formed on a tumor tm5, in the first taken image.

In the embodiment, the hardware structures of processing units, which perform various kinds of processing, such as the signal processing unit 39, the display control unit 40, and the system control unit 41, are various processors to be described later. Various processors include: a central processing unit (CPU) that is a general-purpose processor functioning as various processing units by executing software (program); a programmable logic device (PLD) that is a processor of which the circuit configuration can be changed after the manufacture of a field programmable gate array (FPGA) and the like; a dedicated electrical circuit that is a processor having circuit configuration designed for exclusive use to perform various kinds of processing; and the like.

One processing unit may be formed of one of these various processors, or may be formed of a combination of two or more same kind or different kinds of processors (for example, a plurality of FPGAs or a combination of a CPU and an FPGA). Further, a plurality of processing units may be formed of one processor. As an example where a plurality of processing units are formed of one processor, first, there is an aspect where one processor is formed of a combination of one or more CPUs and software so as to be typified by a computer, such as a client or a server, and functions as a plurality of processing units. Second, there is an aspect where a processor fulfilling the functions of the entire system, which includes a plurality of processing units, by one integrated circuit (IC) chip is used so as to be typified by System On Chip (SoC) or the like. In this way, various processing units are formed using one or more of the above-mentioned various processors as hardware structures.

In addition, the hardware structures of these various processors are more specifically electrical circuitry where circuit elements, such as semiconductor elements, are combined.

Explanation of References

10: endoscope apparatus
12: endoscope
12a: insertion part
12b: operation part
12c: bendable portion
12d: distal end portion
12e: angle knob
13: mode changeover switch
14: light source device
16: processor device
18: monitor
19: user interface
21: objective lens
21A: visual field
21b: outside of range
21B: effective visual field
21C: effective imaging range
21X: range
22: illumination lens
23: auxiliary measurement lens
24: opening
25: air/water supply nozzle
26: light source unit
27: light source control unit
28: light guide
29a: illumination optical system
29b: imaging optical system
30: first auxiliary measurement light-emitting unit
30a: light source
30c: prism
30f: crossing line
30F: plane
32: imaging element
33: imaging control unit
34: circuit
36: communication inter/face (I/F)

38: communication inter/face (I/F)

39: signal processing unit

40: display control unit

41: system control unit

48: imaging sensor

64: display control unit

70A: gradations

70B: frame

80: rising line

82: diagonal line

100: second auxiliary measurement light-emitting unit

101: solid line

102: dotted line

105: spot position recognition unit

107: measurement marker generation unit

107a: marker table

211B: cross section

212A: cross section

212B: cross section

213A: cross section

213B: cross section

**Claims**

1. An endoscope apparatus (10) comprising:

   a light source unit (26) for illumination light adapted to generate illumination light used to illuminate a subject;
   an auxiliary measurement light-emitting unit (30) adapted to emit auxiliary measurement light;
   a light source control unit (27) adapted to allow the illumination light to be continuously emitted and to allow the auxiliary measurement light to be emitted in the form of a pulse at a specific frame interval;
   an imaging element (32) adapted to image the subject;
   a signal processing unit (39) adapted to generate a first taken image obtained through the imaging of the subject illuminated with only the illumination light and generates a second taken image obtained through the imaging of the subject illuminated with the illumination light and the auxiliary measurement light; and
   a display control unit (40); **characterized in that** the display control unit is adapted to allow a display unit to display a specific image where a measurement marker obtained from the second taken image is displayed in the first taken image.

2. The endoscope apparatus according to claim 1,

   wherein the auxiliary measurement light-emitting unit is a first auxiliary measurement light-emitting unit that emits planar auxiliary measurement light as the auxiliary measurement light, and
   the measurement marker is a first measurement marker that includes a crossing line corresponding to a portion, which crosses the subject, of a plane formed by the planar auxiliary measurement light and gradations provided on the crossing line and serving as an index of a size of the subject.

3. The endoscope apparatus according to claim 2, further comprising:

   an imaging optical system that includes an objective lens used to form an image of the subject on the imaging element,
   wherein the auxiliary measurement light-emitting unit emits the auxiliary measurement light in a state where the plane crosses an optical axis of the objective lens, and
   the plane is included in an effective imaging range that is a range where an effective visual field predetermined in a visual field of the imaging optical system and a depth-of-field of the imaging optical system overlap with each other.

4. The endoscope apparatus according to claim 1,

wherein the auxiliary measurement light-emitting unit is a second auxiliary measurement light-emitting unit that emits spot-like auxiliary measurement light as the auxiliary measurement light,

the signal processing unit includes a spot position recognition unit that recognizes a position of a spot, which is a substantially circular area formed on the subject by the spot-like auxiliary measurement light, in the second taken image, and a measurement marker generation unit that includes a second measurement marker representing an actual size of the subject as the measurement marker on the basis of the position of the spot in the second taken image, and

the display control unit displays the second measurement marker in the first taken image.

5. The endoscope apparatus according to claim 4,
wherein the display control unit displays a spot display portion, which corresponds to the position of the spot, in the first taken image in addition to the second measurement marker.

6. The endoscope apparatus according to claim 5,
wherein the second measurement marker has any one of a cruciform shape, a cruciform shape with gradations, a distorted cruciform shape, a circular-and-cruciform shape, or a shape of a measurement point group.

7. The endoscope apparatus according to claim 5,
wherein the second measurement marker has any one of a shape of a plurality of concentric circles, a shape of a plurality of color concentric circles, or a shape of a plurality of distorted concentric circles.

8. The endoscope apparatus according to any one of claims 1 to 7,

wherein the imaging element is a global shutter-type imaging element that performs exposure and reading of electric charges on each pixel at the same timing to output an image signal used to obtain the first taken image or the second taken image, and

until the first taken image is acquired at a second timing next to a first timing after the first taken image is acquired at the first timing, the first taken image acquired at the first timing is continuously displayed and the second taken image is not displayed in the specific image.

9. The endoscope apparatus according to any one of claims 1 to 7,

wherein the imaging element is a rolling shutter-type imaging element that includes a plurality of lines used to image an object to be observed illuminated with the illumination light or the auxiliary measurement light, performs exposure at different exposure timings for the respective lines, and reads electric charges at different reading timings for the respective lines to output an image signal used to obtain the first taken image or the second taken image, and

until the first taken image is acquired at a second timing next to a first timing after the first taken image is acquired at the first timing, the first taken image acquired at the first timing is continuously displayed and the second taken image is not displayed in the specific image.

10. The endoscope apparatus according to any one of claims 1 to 7,

wherein the imaging element is a rolling shutter-type imaging element that includes a plurality of lines used to image an object to be observed illuminated with the illumination light or the auxiliary measurement light, performs exposure at different exposure timings for the respective lines, and reads electric charges at different reading timings for the respective lines to output an image signal used to obtain the first taken image or the second taken image,

the rolling shutter-type imaging element provides a blanking period in which the output of the image signal is prohibited,

the light source control unit emits the auxiliary measurement light in the blanking period, and

until the first taken image is acquired at a second timing next to a first timing after the first taken image is acquired at the first timing, the first taken image acquired at the first timing is continuously displayed and the second taken image is not displayed in the specific image.

11. The endoscope apparatus according to claim 10,

wherein a first mode in which the first taken image is displayed on the display unit and a second mode in which the specific image is displayed on the display unit are provided, and
a reading period of the imaging element in the second mode is set shorter than a reading period of the imaging element in the first mode.

12. The endoscope apparatus according to claim 11,
wherein a frame rate of the display unit, which displays the specific image, is equal to a value of a sum of the reading period of the imaging element in the second mode and the blanking period.

13. The endoscope apparatus according to claim 10,

wherein a first mode in which the first taken image is displayed on the display unit and a second mode in which the specific image is displayed on the display unit are provided, and
a reading period of the imaging element in the first mode is set equal to a reading period of the imaging element in the second mode.

14. The endoscope apparatus according to claim 13,
wherein a value of a sum of the reading period of the imaging element in the first mode and the blanking period is equal to a value of a sum of the reading period of the imaging element in the second mode and the blanking period.


**Patentansprüche**

1. Endoskopvorrichtung (10), umfassend:

eine Lichtquelleneinheit (26) für Beleuchtungslicht, ausgebildet zum Erzeugen von Beleuchtungslicht, das zum Beleuchten eines Subjekts verwendet wird;
eine Hilfsmess-Lichtemissionseinheit (30), ausgebildet zum Emittieren von Hilfsmesslicht;
eine Lichtquellensteuereinheit (27), ausgebildet zum Ermöglichen, dass das Beleuchtungslicht kontinuierlich emittiert wird, und zum Ermöglichen, dass das Hilfsmesslicht in Form eines Impulses in einem spezifischen Vollbildintervall emittiert wird;
ein Bildgebungselement (32), ausgebildet zum Abbilden des Subjekts;
eine Signalverarbeitungseinheit (39), ausgebildet zum Erzeugen eines ersten Aufnahmebilds, gewonnen durch Abbilden des nur mit dem Beleuchtungslicht beleuchteten Subjekts und zum Erzeugen eines zweiten Aufnahmebilds, erhalten durch Abbilden des mit dem Beleuchtungslicht und dem Hilfsmesslicht beleuchteten Subjekts; und
eine Anzeigesteuereinheit (40), **dadurch gekennzeichnet, dass**
die Anzeigesteuereinheit ausgebildet ist, um es einer Anzeigeeinheit zu ermöglichen, ein spezifisches Bild anzuzeigen, bei dem eine Messmarkierung, die aus dem zweiten Aufnahmebild gewonnen wird, in dem ersten Aufnahmebild angezeigt wird.

2. Endoskopvorrichtung nach Anspruch 1,

bei der die Hilfsmesslicht-Emissionseinheit eine erste Hilfsmesslicht-Emissionseinheit ist, die planares Hilfsmesslicht als das Hilfsmesslicht emittiert, und
die Messmarkierung eine erste Messmarkierung ist, die eine querende Linie entsprechend einem Abschnitt enthält, der das Subjekt quert, innerhalb einer Ebene, die durch das planare Hilfsmesslicht gebildet wird, außerdem Gradationen enthält, die sich auf der querenden Linie befinden und als Index für eine Größe des Subjekts dienen.

3. Endoskopvorrichtung nach Anspruch 2, weiterhin umfassend:

eine Abbildungsoptik, die ein Objektiv enthält, das zum **??** eines Bilds auf dem Bildgebungselement verwendet wird,
wobei die Hilfsmesslicht-Emissionseinheit das Hilfsmesslicht in einem Zustand emittiert, in welchem die Ebene eine optische Achse des Objektivs kreuzt, und
die Ebene in einem effektiven Bildgebungsbereich enthalten ist, bei dem es sich um einen Bereich handelt, wo ein effektives Gesichtsfeld, das in einem Gesichtsfeld der Abbildungsoptik vorbestimmt ist, und ein Tiefenbild der Abbildungsoptik einander überlappen.

4. Endoskopvorrichtung nach Anspruch 1,

   bei der die Hilfslicht-Messeinheit eine zweite Hilfsmesslicht-Emissionseinheit ist, die punktförmiges Hilfsmesslicht als das Hilfsmesslicht emittiert,
   die Signalverarbeitungseinheit eine Punktort-Erkennungseinheit enthält, die einen Ort eines Punkts erkennt, bei dem es sich im wesentlichen um eine kreisförmige Fläche handelt, die auf dem Subjekt durch das punktförmige Hilfsmesslicht in dem zweiten Aufnahmebild erzeugt wird, und eine Messmarkierungs-Erzeugungseinheit enthält, die eine zweite Messmarkierung enthält, welche eine aktuelle Größe des Subjekts repräsentiert, und zwar als die Messmarkierung auf der Grundlage des Orts des Punkts in dem zweiten Aufnahmebild, und
   die Anzeigesteuereinheit die zweite Messmarkierung in dem ersten Aufnahmebild anzeigt.

5. Endoskopvorrichtung nach Anspruch 4,
   bei der die Anzeigesteuereinheit einen Punktanzeigeabschnitt, der dem Ort des Punkts entspricht, in dem ersten Aufnahmebild zusätzlich zu der zweiten Messmarkierung anzeigt.

6. Endoskopvorrichtung nach Anspruch 5,
   bei dem die zweite Messmarkierung eine Kreuzform, eine Kreuzform mit Skaleneinteilung, eine verzerrte Kreuzform, eine Kreis- und Kreuzform oder eine Form einer Messpunktgruppe aufweist.

7. Endoskopvorrichtung nach Anspruch 5,
   bei der die zweite Messmarkierung eine Form aus mehreren konzentrischen Kreisen, eine Form aus einer Mehrzahl von farblichen konzentrischen Kreisen oder eine Form aus einer Mehrzahl verzerrter konzentrischer Kreise aufweist.

8. Endoskopvorrichtung nach einem der Ansprüche 1 bis 7,

   bei der das Bildgebungselement ein Bildgebungselement vom Globalverschlusstyp ist, der eine Belichtung und ein Lesen von elektrischen Ladungen in jedem Pixel gleichzeitig ausführt, um ein Bildsignal auszugeben, das dazu dient, das erste Aufnahmebild oder das zweite Aufnahmebild zu gewinnen, und
   bis das erste Aufnahmebild zu einer zweiten Zeit nächst einer ersten Zeit erfasst wird, nachdem das erste Aufnahmebild zu der ersten Zeit erfasst wurde, das erste Aufnahmebild, das zu der ersten Zeit erfasst wurde, kontinuierlich dargestellt wird und das zweite Aufnahmebild in dem spezifischen Bild nicht angezeigt wird.

9. Endoskopvorrichtung nach einem der Ansprüche 1 bis 7,

   bei der das Bildgebungselement ein Bildgebungselement vom Rollverschlusstyp ist, das mehrere Zeilen aufweist, um ein zu beobachtendes und mit dem Beleuchtungslicht oder dem Hilfsmesslicht beleuchtetes Objekt abzubilden, eine Belichtung zu unterschiedlichen Belichtungszeiten für die jeweiligen Zeilen ausführt, und elektrische Ladungen zu unterschiedlichen Lesezeiten für die jeweiligen Zeilen ausliest, um ein Bildsignal auszugeben, das zum Gewinnen des ersten Aufnahmebilds oder des zweiten Aufnahmebilds dient, und
   bis das erste Aufnahmebild zu einer zweiten Zeit nächst einer ersten Zeit erfasst wird, nachdem das erste Aufnahmebild zu der ersten Zeit erfasst wurde, das erste Aufnahmebild, das zu der ersten Zeit erfasst wurde, kontinuierlich dargestellt wird und das zweite Aufnahmebild in dem spezifischen Bild nicht angezeigt wird.

10. Endoskopvorrichtung nach einem der Ansprüche 1 bis 7,

    bei der das Bildgebungselement ein Bildgebungselement vom Rollverschlusstyp ist, das mehrere Zeilen aufweist, um ein zu beobachtendes und mit dem Beleuchtungslicht oder dem Hilfsmesslicht beleuchtetes Objekt abzubilden, eine Belichtung zu unterschiedlichen Belichtungszeiten für die jeweiligen Zeilen ausführt, und elektrische Ladungen zu unterschiedlichen Lesezeiten für die jeweiligen Zeilen ausliest, um ein Bildsignal auszugeben, das zum Gewinnen des ersten Aufnahmebilds oder des zweiten Aufnahmebilds dient, und
    wobei das Bildgebungselement vom Rollverschlusstyp eine Austastperiode bereitstellt, in welcher die Ausgabe des Bildsignals unterbunden ist,
    die Lichtquellensteuereinheit das Hilfsmesslicht in der Austastperiode emittiert, und
    bis das erste Aufnahmebild zu einer zweiten Zeit nächst einer ersten Zeit erfasst wird, nachdem das erste Aufnahmebild zu der ersten Zeit erfasst wurde, das erste Aufnahmebild, das zu der ersten Zeit erfasst wurde, kontinuierlich dargestellt wird und das zweite Aufnahmebild in dem spezifischen Bild nicht angezeigt wird.

11. Endoskopvorrichtung nach Anspruch 10,

bei der eine erste Betriebsart, in der das erste Aufnahmebild auf der Anzeigeeinheit dargestellt wird, und eine zweite Betriebsart, in der das spezifische Bild auf der Anzeigeeinheit dargestellt wird, vorhanden sind, und eine Lesezeitspanne des Bildgebungselements in der zweiten Betriebsart kürzer eingestellt ist als eine Lesezeitspanne des Bildgebungselements in der ersten Betriebsart.

**12.** Endoskopvorrichtung nach Anspruch 11,
bei der eine Bildwiederholrate der Anzeigeeinheit, die das spezifische Bild anzeigt, gleich einem Wert ist, der einer Summe der Lesezeitspanne des Bildgebungselements in der zweiten Betriebsart und der Austastzeitspanne entspricht.

**13.** Endoskopvorrichtung nach Anspruch 10,

bei der eine erste Betriebsart, in der das erste Aufnahmebild auf der Anzeigeeinheit dargestellt wird, und eine zweite Betriebsart, in der das spezifische Bild auf der Anzeigeeinheit dargestellt wird, vorhanden sind, und eine Lesezeitspanne des Bildgebungselements in der ersten Betriebsart auf den gleichen Wert eingestellt ist wie eine Lesezeitspanne des Bildgebungselements in der zweiten Betriebsart.

**14.** Endoskopvorrichtung nach Anspruch 13,
bei der ein Wert einer Summe aus der Lesezeitspanne des Bildelements in der ersten Betriebsart und die Austastzeitspanne gleich ist einem Wert einer Summe aus der Lesezeitspanne des Bildgebungselements in der zweiten Betriebsart und der Austastzeitspanne.

**Revendications**

**1.** Appareil endoscopique (10), comprenant :

une unité formant source lumineuse (26) pour lumière d'éclairage, apte à générer une lumière d'éclairage utilisée pour éclairer un sujet ;
une unité d'émission lumineuse de mesure auxiliaire (30), apte à émettre une lumière de mesure auxiliaire ;
une unité de commande de source lumineuse (27), apte à permettre une émission continue de la lumière d'éclairage et à permettre une émission de la lumière de mesure auxiliaire sous la forme d'une impulsion sur un intervalle de trame spécifique ;
un élément d'imagerie (32), apte à imager le sujet ;
une unité de traitement de signaux (39) apte à générer une première image prise, obtenue en imageant le sujet éclairé avec uniquement la lumière d'éclairage, et à générer une seconde image prise, obtenue en imageant le sujet éclairé avec la lumière d'éclairage et la lumière de mesure auxiliaire, et
une unité de commande d'affichage (40) ;
**caractérisé en ce que** l'unité de commande d'affichage est apte à permettre à une unité d'affichage d'afficher une image spécifique où un marqueur de mesure obtenu à partir de la seconde image prise est affiché dans la première image prise.

**2.** Appareil endoscopique selon la revendication 1,

dans lequel l'unité d'émission lumineuse de mesure auxiliaire est une première unité d'émission lumineuse de mesure auxiliaire, laquelle émet une lumière de mesure auxiliaire planaire comme lumière de mesure auxiliaire, et
le marqueur de mesure est un premier marqueur de mesure, lequel inclut une ligne de croisement correspondant à une portion, laquelle croise le sujet, d'un plan formé par la lumière de mesure auxiliaire planaire et des gradations fournies sur la ligne de croisement comme indice d'une taille du sujet.

**3.** Appareil endoscopique selon la revendication 2, comprenant en outre

un système optique d'imagerie, lequel inclut une lentille de focalisation utilisée pour former une image du sujet sur l'élément d'imagerie,
dans lequel l'unité d'émission lumineuse de mesure auxiliaire émet la lumière de mesure auxiliaire dans un état où le plan croise un axe optique de la lentille de focalisation, et
le plan est inclus dans une plage d'imagerie effective, laquelle est une plage où un champ visuel effectif prédéterminé dans un champ visuel du système optique d'imagerie et une profondeur de champ du système optique d'imagerie se chevauchent entre eux.

4. Appareil endoscopique selon la revendication 1,

dans lequel l'unité d'émission lumineuse de mesure auxiliaire est une seconde unité d'émission lumineuse de mesure auxiliaire, laquelle émet une lumière de mesure auxiliaire similaire à un point lumineux comme lumière de mesure auxiliaire ;

l'unité de traitement de signaux inclut une unité de reconnaissance de position de point lumineux, laquelle reconnaît une position d'un point lumineux, laquelle est une zone en grande partie circulaire formée sur le sujet par la lumière de mesure auxiliaire similaire à un point lumineux, dans la seconde image prise, et une unité de génération de marqueur de mesure, laquelle inclut un second marqueur de mesure représentant une taille réelle du sujet comme marqueur de mesure sur la base de la position du point lumineux dans la seconde image prise, et

l'unité de commande d'affichage affiche le second marqueur de mesure dans la première image prise.

5. Appareil endoscopique selon la revendication 4,
dans lequel l'unité de commande d'affichage affiche une portion d'affichage de point lumineux, laquelle correspond à la position du point lumineux, dans la première image prise, outre le second marqueur de mesure.

6. Appareil endoscopique selon la revendication 5,
dans lequel le second marqueur de mesure présente l'une quelconque des formes parmi une forme cruciforme, une forme cruciforme avec gradations, une forme cruciforme déformée, ou une forme circulaire et cruciforme, ou une forme d'un groupe de points de mesure.

7. Appareil endoscopique selon la revendication 5,
dans lequel le second marqueur de mesure présente l'une quelconque des formes parmi une pluralité de cercles concentriques, une forme d'une pluralité de cercles concentriques de couleur, ou une forme d'une pluralité de cercles concentriques déformés.

8. Appareil endoscopique selon l'une quelconque des revendications 1 à 7,

dans lequel l'élément d'imagerie est un élément d'imagerie de type à obturateur global, lequel réalise une exposition et lecture de charges électriques sur chaque pixel sur la même temporisation afin de produire un signal d'image utilisé pour obtenir la première image prise ou la seconde image prise, et

jusqu'à ce que la première image prise soit acquise sur une seconde temporisation adjacente à une première temporisation après que la première image prise est acquise sur la première temporisation, la première image prise acquise sur la première temporisation est affichée de manière continue, et la seconde image prise n'est pas affichée dans l'image spécifique.

9. Appareil endoscopique selon l'une quelconque des revendications 1 à 7,

dans lequel l'élément d'imagerie est un élément d'imagerie de type à obturateur roulant, lequel inclut une pluralité de lignes utilisées pour imager un sujet devant être observé, éclairé avec la lumière d'éclairage ou la lumière de mesure auxiliaire, réalise l'exposition sur des temporisations d'exposition différentes pour les lignes respectives, et lit des charges électriques sur des temporisations de lecture différentes pour les lignes respectives afin de produire un signal d'image utilisé pour obtenir la première image prise ou la seconde image prise, et

jusqu'à ce que la première image prise soit acquise sur une seconde temporisation adjacente à une première temporisation après que la première image prise est acquise sur la première temporisation, la première image prise acquise sur la première temporisation est affichée de manière continue, et la seconde image prise n'est pas affichée dans l'image spécifique.

10. Appareil endoscopique selon l'une quelconque des revendications 1 à 7,

dans lequel l'élément d'imagerie est un élément d'imagerie de type à obturateur roulant, lequel inclut une pluralité de lignes utilisées pour imager un sujet devant être observé, éclairé avec la lumière d'éclairage ou la lumière de mesure auxiliaire, réalise l'exposition sur des temporisations d'exposition différentes pour les lignes respectives, et lit des charges électriques sur des temporisations de lecture différentes pour les lignes respectives afin de produire un signal d'image utilisé pour obtenir la première image prise ou la seconde image prise ;

l'élément d'imagerie de type à obturateur roulant fournit une période de suppression, où la production du signal d'image est empêchée ;

l'unité de commande de source lumineuse émet la lumière de mesure auxiliaire dans la période de suppression, et

jusqu'à ce que la première image prise soit acquise sur une seconde temporisation adjacente à une première temporisation après que la première image prise est acquise sur la première temporisation, la première image prise acquise sur la première temporisation est affichée de manière continue, et la seconde image prise n'est pas affichée dans l'image spécifique.

11. Appareil endoscopique selon la revendication 10,

dans lequel un premier mode, où la première image prise est affichée sur l'unité d'affichage, et un second mode, où l'image spécifique est affichée sur l'unité d'affichage, sont fournis, et

une période de lecture de l'élément d'imagerie dans le second mode est réglée pour être plus courte qu'une période de lecture de l'élément d'imagerie dans le premier mode.

12. Appareil endoscopique selon la revendication 11,

dans lequel une fréquence d'images de l'unité d'affichage, laquelle affiche l'image spécifique, est égale à une valeur d'une somme de la période de lecture de l'élément d'imagerie dans le second mode et la période de suppression.

13. Appareil endoscopique selon la revendication 10,

dans lequel un premier mode, où la première image prise est affichée sur l'unité d'affichage, et un second mode, où l'image spécifique est affichée sur l'unité d'affichage sont fournis, et

une période de lecture de l'élément d'imagerie dans le premier mode est réglée pour être égale à une période de lecture de l'élément d'imagerie dans le second mode.

14. Appareil endoscopique selon la revendication 13,

dans lequel une valeur d'une somme de la période de lecture de l'élément d'imagerie dans le premier mode et de la période de suppression est égale à une valeur d'une somme de la période de lecture de l'élément d'imagerie dans le second mode et de la période de suppression.

## FIG. 1

EP 3 571 976 B1

# FIG. 2

FIG. 3

## FIG. 4

Lm

23

30

FIRST AUXILIARY MEASUREMENT
LIGHT-EMITTING UNIT

30c    30b    30a

DOE ← LIGHT
SOURCE

## FIG. 5

Lm

R1

21A

L1

21B

12d

Ax

D1

21C

21

30

23

P3    P2    P1

D3

## FIG. 6

## FIG. 7

## FIG. 8

## FIG. 9

## FIG. 10

## FIG. 11

## FIG. 12

## FIG. 13

FIG. 14

FIG. 15

## FIG. 16

## FIG. 17

| LIGHT-EMITTING FRAME | FLx | FLy | FLx | FLy | FLx | FLy |
|---|---|---|---|---|---|---|

ILLUMINATION LIGHT on/off

AUXILIARY MEASUREMENT LIGHT on/off

## FIG. 18

NORMAL MODE | LENGTH MEASUREMENT MODE

CCD (FRAME PERIOD)
GLOBAL SHUTTER

80  80  80  80  80  80  80

1 FRAME

ILLUMINATION
LIGHT — on / off

AUXILIARY
MEASUREMENT
LIGHT — on / off

IMAGING ELEMENT
OUTPUT

| N | N + Lm | N | N + Lm | N | N + Lm | N |

DISPLAY IMAGE
OUTPUT

| N | S | S | S | S | S | S |

TIMING  T1  T2  T3  T4  T5  T6  T7  T8

## FIG. 19

NORMAL MODE | LENGTH MEASUREMENT MODE

Ls

CMOS (FRAME PERIOD)
ROLLING SHUTTER

1 FRAME

82 Lt  82  82  82  82  82  82  82

ILLUMINATION
LIGHT — on / off

AUXILIARY
MEASUREMENT
LIGHT — on / off

IMAGING ELEMENT
OUTPUT

| N | N + Lm | N + Lm | N | N + Lm | N + Lm | N |

DISPLAY IMAGE
OUTPUT

| N | S | S | S | S | S | S |

TIMING  T1  T2  T3  T4  T5  T6  T7  T8

33

# FIG. 20

EP 3 571 976 B1

# FIG. 21

EP 3 571 976 B1

# FIG. 22

TIMING: T1, T2, T3, T4, T5, T6, T7, T8

NORMAL MODE | LENGTH MEASUREMENT MODE

Ls | 1 FRAME | Lt

CMOS (FRAME PERIOD) ROLLING SHUTTER: 82, 82, 82, 82, 82, 82, 82, 82

Prc | Prs | Bk | Bk | Bk | Bk | Bk | Bk | Bk

ILLUMINATION LIGHT: on/off

AUXILIARY MEASUREMENT LIGHT: on/off

IMAGING ELEMENT OUTPUT: N | N | N + Lm | N | N + Lm | N | N + Lm | N

DISPLAY IMAGE OUTPUT: N | S | S | S | S | S | S

EP 3 571 976 B1

## FIG. 23

Ln

23

100

SECOND AUXILIARY MEASUREMENT
LIGHT-EMITTING UNIT

30c          30a

LIGHT SOURCE
(LASER LIGHT
SOURCE MODULE)

## FIG. 24

101

Rx

102

Qz          Qy

Qx

12d

Ax

D1          Ln

21

102

23          100

101

Pz          Py          Px

D3

# FIG. 25

39

SIGNAL PROCESSING UNIT

105

SPOT POSITION RECOGNITION
UNIT

107

MEASUREMENT MARKER
GENERATION UNIT

MARKER TABLE
107a

# FIG. 26

M1

tm1

SP1

## FIG. 27

## FIG. 28

## FIG. 29

| CRUCIFORM SHAPE WITH GRADATIONS | DISTORTED CRUCIFORM SHAPE | CIRCULAR-AND -CRUCIFORM SHAPE | SHAPE OF MEASUREMENT POINT GROUP |
|---|---|---|---|

## FIG. 30

# FIG. 31

# FIG. 32

x (PIXEL POSITION OF SPOT IN X DIRECTION)

## FIG. 33

y (PIXEL POSITION OF SPOT IN Y DIRECTION)

## FIG. 34

x (PIXEL POSITION OF SPOT IN X DIRECTION)

## FIG. 35

## FIG. 36

## FIG. 37

## FIG. 38

**REFERENCES CITED IN THE DESCRIPTION**

**Patent documents cited in the description**

- JP 4012724 A
- JP H04012724 A
- JP 2017508529 A
- WO 2018051679 A1